# FASCICULE DE BREVET EUROPEEN

(11) **EP 1 202 992 B1**
(45) Date de publication et mention de la délivrance du brevet: **15.09.2004**
(21) Numéro de dépôt: 00958678.5
(22) Date de dépôt: 11.08.2000
(51) Int. Cl.: C07D 471/16, A61K 31/4375, A61P 35/00

(54) **DERIVES D'ASCIDIDEMINE ET LEURS APPLICATIONS THERAPEUTIQUES**
ASCIDIDEMINDERIVATE UND IHRE THERAPEUTISCHE VERWENDUNG
ASCIDIDEMIN DERIVATIVES AND THEIR THERAPEUTIC APPLICATIONS

(30) Priorité: 13.08.1999 FR 9910490; 24.05.2000 FR 0006652
(43) Date de publication de la demande: 08.05.2002
(73) Titulaire: CEPHALON FRANCE, 94700 Maisons-Alfort (FR)
(72) Inventeur: DELFOURNE, Evelyne, F-66450 Pollestres (FR); DARRO, Francis, 1070 Bruxelles (BE); BASTIDE, Jean, F-66000 Perpignan (FR); KISS, Robert, B-1440 Wauthier-Braine (BE); FRYDMAN, Armand, F-91370 Verrières-le-Buisson (FR)
(74) Mandataire: Bernasconi, Jean
(86) Numéro de dépôt international: PCT/FR2000/002312
(87) Numéro de publication internationale: WO 2001/012631

(56) Documents cités:
- SCHMITZ F J ET AL: "Biologically active compounds from marine organisms" PURE & APPLIED CHEMISTRY,GB,PERGAMON PRESS, OXFORD, vol. 7, no. 62, 1990, pages 1393-1396, XP002074302 ISSN: 0033-4545 cité dans la demande
- BRACHER F.: "Polyciclic aromatic alkaloids" PHARMAZIE., vol. 52, no. 1, 1997, pages 57-60, XP002136779 VEB VERLAG VOLK UND GESUNDHEIT. BERLIN., DD ISSN: 0031-7144 cité dans la demande
- LINDSAY B S ET AL: "STRUCTURAL REQUIREMENTS FOR BIOLOGICAL ACTIVITY OF THE MARINE ALKALOID ASCIDIDEMIN" BIOORGANIC & MEDICINAL CHEMISTRY LETTERS,GB,OXFORD, vol. 5, no. 7, 1 janvier 1995 (1995-01-01), pages 739-742, XP002036209 ISSN: 0960-894X cité dans la demande

## Description

La présente invention concerne des compositions pharmaceutiques à base de composés polyaromatiques utiles notamment comme médicaments antitumoraux.

En 1999, les traitements cytotoxiques (chimiothérapie) utilisés pour réduire la taille des tumeurs cancéreuses, contenir le développement du processus tumoral voire, dans trop peu de cas encore, supprimer les amas de cellules cancéreuses et le risque de métastases, combinent des substances chimiques d'introduction récente avec d'autres qui sont utilisées depuis quelques dizaines d'années. Par exemple, au 5-fluorouracil (5-FU), reconnu depuis près de 40 ans comme l'un des traitements les plus actifs du cancer colo-rectal, peut être substitué l'un ou l'autre des inhibiteurs spécifiques de la topoisomérase I (irinotécan ou topotécan) lorsque la tumeur n'est plus sensible au 5-FU. Plus généralement, l'arsenal thérapeutique disponible pour traiter les tumeurs colo-rectales va également s'enrichir avec la mise à disposition de l'oxaliplatine, des nouveaux "donneurs" in situ de 5-FU ou des inhibiteurs sélectifs de la thymidylate synthétase. Cette coexistence ne se limite pas au traitement des cancers colo-rectaux puisque, également, la chimiothérapie des cancers du sein, de l'ovaire, du poumon fait maintenant largement appel à la famille des dérivés des taxanes (paclitaxel, docetaxel). Le besoin de traitements plus efficaces et mieux tolérés, améliorant ainsi la survie et la qualité de vie des malades est impérieux puisque, en prenant toujours l'exemple des tumeurs colo-rectales, il a été estimé (S.L. Parker, T. Tong, S. Bolden *et al*., CA Cancer J. Clin., 1997) que, rien qu'aux Etats-Unis plus de 131 000 nouveaux cas ont été diagnostiqués en 1997, dont 54 000 étaient responsables du décès des patients. C'est la connaissance de cette situation qui a incité les inventeurs à s'intéresser à une famille de composés polyaromatiques encore peu étudiés, identifiés chez des Ascidies de mers chaudes, pour développer une chimie médicinale originale destinée à sélectionner des composés synthétiques issus d'un travail de conception/modulation chimique et doués d'une activité cytotoxique significative au plan thérapeutique.

Les mers et les océans qui couvrent plus de 70 % de la surface du globe, hébergent des plantes marines et des éponges dont l'étude pharmacognosique systématique progressive montre que ces espèces vivantes peuvent contenir des alcaloïdes complexes présentant des propriétés pharmacologiques intéressantes.

Par exemple, les éponges *Cryptotheca crypta* et *Halichondria okadai* font l'objet d'études approfondies depuis la découverte de la présence, dans leurs cellules, de cytarabine ou d'halichondrine B. Il en est de même pour la famille des tuniciers, depuis l'isolement de l'aplidine du tunicier *Aplidium albicans* qui vit dans les îles Baléares (Espagne). Des alcaloïdes à structure tétrahydroisoquinolone ont été isolés de l'ascidie *Ecteinascidia turbinata*. Parmi ceux-ci, l'ecteinascidin-743 fait l'objet de travaux pré-cliniques approfondis (E. Igbicka *et al*., NCI-EORTC symposium, 1998; Abst. 130 p.34), ainsi que d'essais cliniques destinés à définir son potentiel thérapeutique comme médicament anticancéreux (A. Bowman *et al*., NCI-EORTC symposium, 1998; Abst. 452 p.118 ; M.Villanova-Calero *et al*., NCI-EORTC symposium, 1998; Abst. 453 p.118 ; M.J.X. Hillebrand *et al*., NCI-EORTC symposium, 1998; Abst. 455 p.119; E. Citkovic *et al*., NCI-EORTC symposium, 1998; Abst. 456 p.119). De nouveaux dérivés d'acridines pentacycliques font également l'objet de travaux de pharmaco-chimie (D.J. Hagan *et al*., J. Chem. Soc., Perkin Transf., 1997; 1: 2739-2746).

Autre alcaloïde naturel d'origine marine, l'ascididémine a été extraite du tunicier *Didemnum sp*. (J. Kobayashi *et al*., Tehahedron, lett. 1988 ; 29 : 1177-80) et de l'ascidie *Cystodytes dellechiajei* (I. Bonnard *et al*, Anti-cancer Drug design 1995 ; 10 : 333-46). L'ascididémine possède des propriétés antiprolifératives mises en évidence sur le modèle de leucémie murine (lignées P388 ou L1210) et décrites par F. Schmitz *et al*. (J. Org. Chem. 1991 ; 56 : 804-8), B. Lindsay *et al*. (Bioorg. Med. Chem. Lett. 1995 ; 5 : 739-42) et J. Kobayashi *et al*. (Tehahedron lett. 1988 ; 29 : 1177-80) et sur le modèle de leucémie humaine décrites par I. Bonnard *et al*. (Anti-cancer Drug design 1995 ; 10 : 333-46). Plusieurs voies de synthèse de l'ascididémine ont été rapportées par différents auteurs : F. Bracher *et al*. (Heterocycles 1989 ; 29 : 2093-95), C.J. Moody *et al.* (Tetrahedron Lett. 1992 ; 48 : 3589-602) et G. Gellerman *et al*. (Synthesis 1994 ; 239-41).

On peut également citer la 2-bromoleptoclinidone (selon la dénomination de S.J. Bloor *et al*. 1987) et isolée de l'ascidie *Leptoclinides sp*. par S.J. Bloor *et al*. (J. Ann. Chem. Soc. 1987 ; 109 : 6134-6) et synthétisée par F. Bracher *et al*. (Hétérocycles 1989 ; 29 : 2093-95) puis par M.E. Jung *et al*. (Hétérocycles 1994 ; 39 ; 2 : 767-778). La 2-bromoleptoclinidone présente une cytoxicité sur le modèle cellulaire de leucémie avec une DE 50 de 0.4 µg/ml. Les propriétés cytotoxiques ont été confirmées, par F. Bracher (Pharmazie 1997 ; 52 : 57-60) aussi bien *in vitro* - sur soixante lignées cellulaires tumorales en culture - que *in vivo* sur les modèles de xénogreffes de lignées cellulaires tumorales humaines (tumeurs du colon SW-620 et HTC116, tumeur rénale A498 et mélanome LOX IM VI) implantées chez des souris.

D'autres composés dérivés dé l'ascididémine tels que la 11-hydroxy ascididémine, la 11-méthoxy ascididémine, les 11-phényle et 11-nitrophényle ascididémines, les 1-nitro et 3-nitro ascididémines et la néocalliactine ont été décrits au plan chimique (selon la numérotation de S.J. Bloor *et al*. 1987) par différentes équipes telles que celles de F.J. Schmitz (J. Org. Chem. 1991 ; 56 : 804-8) et de Y. Kitahara *et al*. (Heterocycles 1993 ; 36 : 943-46 ; Tetrahedron Lett. 1997 ; 53, 17029-38), G. Gellerman *et al*. (Tetrahedron lett. 1993 ; 34 : 1827-30), S. Nakahara *et al* (Heterocycles 1993; 36 : 1139-44), I. Spector *et al*. (US Patent Number : 5,432,172, Jul. 11, 1995).

L'ascididémine, la 2-bromoleptoclinidone et l'ascididémine substituée par un groupe hydroxy en position 11 selon la nomenclature employée par l'auteur ont été rapportées par Schmitz et al. (Pure and Applied Chem. 1990, 7(62), 1393-1396) comme présentant une activité cytotoxique *in vitro* vis-à-vis des cellules de leucémie murine (P388).

La présente invention à pour objet une composition pharmaceutique comprenant une quantité efficace d'un composé choisi parmi les composés de formules générales I et la suivantes : 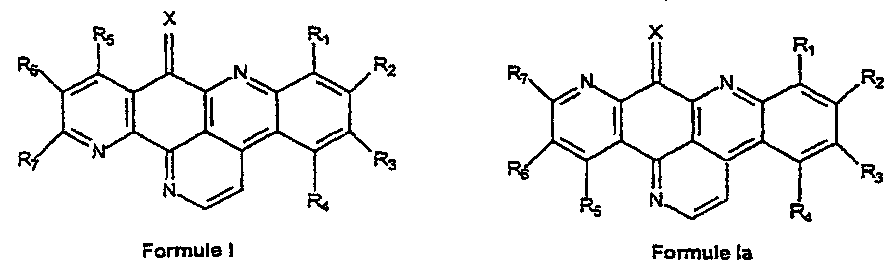 dans lesquelles :
- X est choisi parmi l'oxygène, le groupe =NH, le groupe = N-OH,
- R₁ est choisi parmi l'hydrogène, les halogènes, le groupe nitro et les groupes - NR₈R₉ dans lesquels R₈ et R₉ sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi . l'hydrogène, les groupes alkyle (C₁-C₄), phénylalkyle (C₁-C₄), et les groupes -(CH₂)ₙ-Y avec Y est choisi parmi les halogènes et les groupes CN, -CH(O-Et)₂, alcoxy(C₁-C₆), -O-(CH₂)₂-N(CH₃)₂, -N(CH₃)₂ et n = 1 à 3,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
   l'hydrogène, un atome d'halogène,
   les groupes alkyle en C₁-C₆, hydroxyle, alcoxy en C₁-C₆, alcoxy (C₁-C₆)alkyle(C₁-C₆), alkyl(C₁-C₄) carbonyloxyalkyle(C₁-C₄), -CHO, -COOH, -CN, -CO₂R₁₄, -CONHR₁₄, -CONR₁₄R₁₅, les groupes -NHCOR₁₄ et -NR₁₄R_{15,} dans lesquels R₁₄ et R₁₅ sont choisis indépendamment l'un de l'autre parmi l'hydrogène, les groupes alkyle (C₁-C₆),-phényle-CO-CH₃ et -CH₂-CH₂-N(CH₃)₂,
   les groupes -phényle-CO-CH₃ ou -phényle-CO-CH=CH-N(CH₃)₂, morpholino, nitro, SO₃H,
   les groupes : 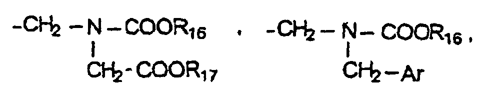 R₁₆ et R₁₇ étant choisis parmi les groupes alkyle en C₁-C₆ et Ar étant ùn groupe aryle en C₆-C₁₄,
et les sels d'addition, de ces composés avec des acides pharmaceutiquement acceptables.

La présente invention a plus particulièrement pour objet une composition pharmaceutique comprenant une quantité efficace d'un composé choisi parmi les composés de formule I dans laquelle :
- X est choisi parmi l'oxygène, le groupe =NH, le groupe = N-OH,
- R₁ est choisi parmi l'hydrogène, les halogènes, le groupe nitro et les groupes - NR₈R₉ dans lesquels R₈ et Rg sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ ,sont choisis, indépendamment l'un de l'autre, parmi l'hydrogène, les groupes alkyle (C₁-C₄), phénylalkyle (C₁-C₄), -(CH₂)₂-N(CH₃)₂, et -(CH₂)₂-O-(CH₂)₂-N(CH₃)₂,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
   l'hydrogène, un atome d'halogène,
   les groupes alkyle en C₁-C₆, hydroxyle, alcoxy en C₁-C₆, -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis indépendamment l'un de l'autre parmi l'hydrogène, les groupes alkyle (C₁-C₆) et -CH₂-CH₂-N(CH₃)₂,
   les groupes -phényle-CO-CH₃ ou -phényle-CO-CH=CH-N(CH₃)₂, morpholino, nitro, SO₃H,
   les groupes : 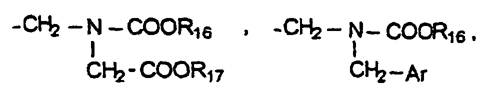 R₁₆ et R₁₇ étant choisis parmi les groupes alkyle en C₁-C₆ et Ar étant un groupe aryle en C₆-C₁₄,
et les sels d'addition de ces composés avec des acides pharmaceutiquement acceptables.

La présente invention a plus particulièrement pour objet une composition pharmaceutique comprenant une quantité efficace d'un composé choisi parmi les composés de formule 1 dans laquelle :
- X représente l'oxygène,
- R₁ est choisi parmi l'hydrogène et le groupe amino,
- R₂ est choisi parmi l'hydrogène et les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes méthyle, phénylalkyle (C₁-C₄), -(CH₂)₂-N(CH₃)₂, -(CH₂)₂-O-(CH₂)₂-N(CH₃)₂;
- R₄ est choisi parmi l'hydrogène, les halogènes et le groupe nitro et amino,
- R₅, R₆ R₇ représentent un hydrogène,
et leurs sels d'addition de ces composés avec des acides pharmaceutiquement acceptables.

Dans sa forme préférée, la présente invention a plus particulièrement pour objet une composition pharmaceutique comprenant une quantité efficace d'un composé choisi parmi les composés de formules I et la dans lesquelles :
- X représente l'oxygène,
- R₁ est choisi parmi l'hydrogène et le groupe amino,
- R₂ est choisi parmi l'hydrogène et les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes méthyle, phénylalkyle (C₁-C₄) et les groupes -(CH₂)ₙ-Y avec Y est choisi parmi les halogènes et les groupes CN, -CH(O-Et)₂, alcoxy(C₁-C₆), -O-(CH₂)₂-N(CH₃)₂, -N(CH₃)₂ et n = 1 à 3,
- R₄ est choisi parmi l'hydrogène, les halogènes et les groupes nitro et amino,
- R₅ est choisi parmi un hydrogène, un halogène ou un groupe méthoxy,
- R₆, R₇ sont choisis parmi l'hydrogène, les groupes alcoxy(C₁-C₆), alcoxy (C₁-C₆)alkyle(C₁-C₆) et -CH₂OCOCH₃,
et les sels d'addition de ces composés avec des acides pharmaceutiquement acceptables.

La présente invention a également pour objet les composés de formule la telle que définie précédemment
et les sels d'addition de ces composés avec des acides pharmaceutiquement acceptables.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques.

De manière générale, les composés de formule I sont obtenus selon le schéma réactionnel général décrit par F. Bracher *et al* (Heterocycles 1989 ; 29 : 2093-95) pour l'ascididémine. Selon ce schéma, les composés sont préparés par amination oxydative d'une 5,8-quinone substituée avec une ortho-aminoacétophénone substituée, suivi de la cyclisation de la diaryl amine obtenue (composés de formule II) en quinone tétracyclique intermédiaire (composés de formule III). L'énamine formée par réaction du composé de formule III avec le diéthyl acétal du diméthylformamide conduit au dérivé final par cyclisation :

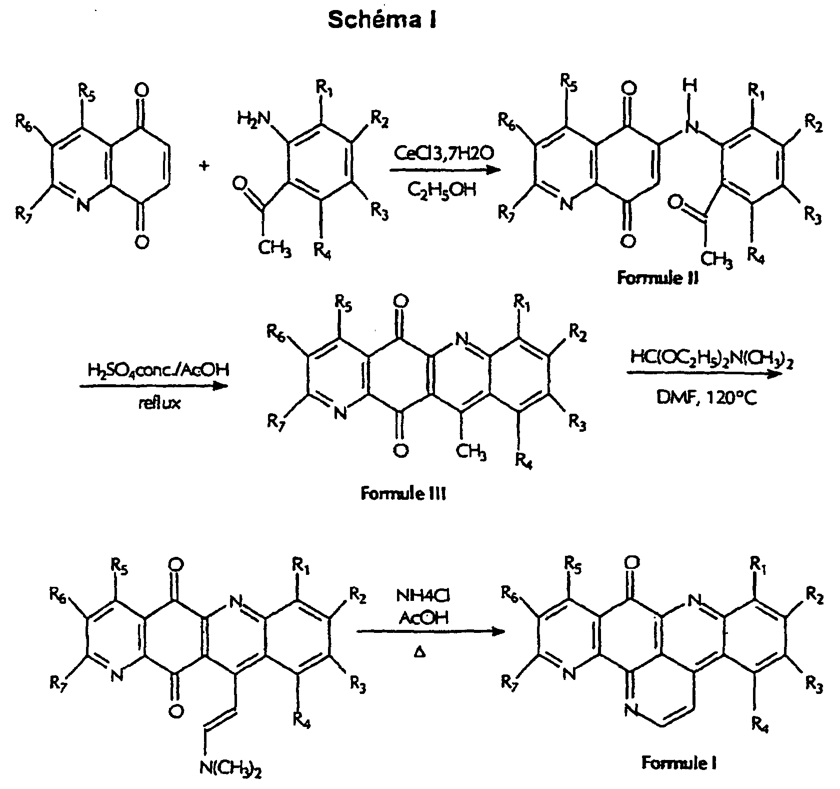

L'ascididémine (ou 9-*H*-quino[4,3,2-*de*][1,10]phénanthrolin-9-one) a été préparée selon le procédé décrit par F. Bracher *et al*. (Heterocycles 1989 ; 29 : 2093-95) et est référencée, dans le présent document, sous le numéro CRL 8274.

Certains composés peuvent être préparés directement à partir de l'ascididémine ou à partir d'un composé de formule I utilisé comme intermédiaire de synthèse.

C'est ainsi en particulier que les composés de formule I dans lesquels R₃ est un groupe -NR₁₀R₁₁, avec R₁₀ et/ou R₁₁ différents de l'hydrogène, peuvent être obtenus à partir d'un composé de formule I dans laquelle R₃ est un groupe -NH₂.

De même, les composés de formule la peuvent être obtenus selon le schéma réactionnel général II. Selon ce schéma, les composés sont préparés par condensation d'un acide chlorobenzoïque substitué et de la diméthoxyaniline pour former les composés de formule IIa. Après transformation de la fonction acide en méthylcétone, cyclisation puis oxydation, une quinone tricyclique intermédiaire (composé de formule IIIa) est obtenue. Une cycloaddition de Diels Alder avec un 1-azadiène conduit à la formation d'une quinone tétracyclique (composé de formule IVa). L'addition du diéthylacétal de diméthylformamide sur cette quinone conduit à un intermédiaire énamine qui se cyclise, en présence de chlorure d'ammonium, en composé final de formule la.

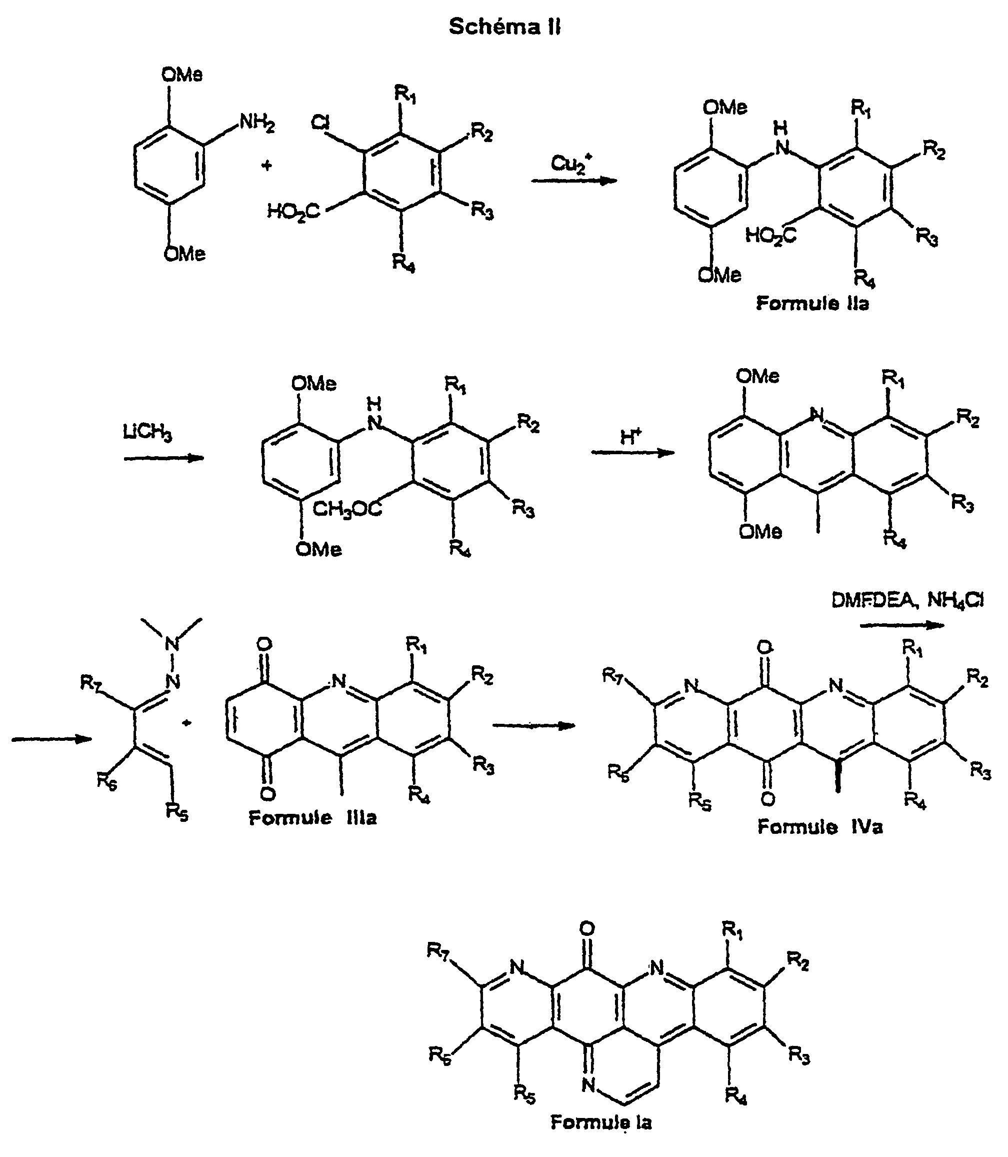

Certains composés peuvent être préparés directement à partir de l'isomère de l'ascididémine appelé 9-*H*-quino[4,3,2-*de*][1,7]phénanthrolin-9-one, ou à partir d'un composé de formule la utilisé comme intermédiaire de synthèse.

Certains composés peuvent être préparés directement à partir de l'isomère de l'ascididémine appelé 9-*H*-quino[4,3,2-*de*][1,7]phénanthrolin-9-one, ou à partir d'un composé de formule la utilisé comme intermédiaire de synthèse.

C'est ainsi en particulier que les composés de formule la dans lesquels R₃ est un groupe -NR₁₀R₁₁, avec R₁₀ et/ou R₁₁ différents de l'hydrogène, peuvent être obtenus à partir d'un composé de formule la dans laquelle R₃ est un groupe - NH₂.

### A - Préparation des produits intermédiaires de formule II (Schéma I)

### A-1 - Synthèse de la 6-(2-acétyl-4-méthyl-phénylamino)-quinoline-5,8-dione (intermédiaire A₁) (CRL 8322)

On additionne lentement une solution de quinoline-5,8-dione (0,215 g, 1,35 mmol) dans 12 ml d'éthanol, à une solution de chlorure de cérium (1g, 2,7 mmol) et de 5-méthyl-2 amino acétophénone (0,402 g, 2,7 mmol) dans 5 ml d'éthanol. Le milieu réactionnel (rouge) est laissé sous agitation à température ambiante une nuit. On hydrolyse par 30 ml d'une solution aqueuse d'acide acétique à 10 % et on extrait 4 fois au chloroforme. Les phases organiques sont séchées sur MgSO₄ puis évaporées. Le brut obtenu est purifié par flash-chromatographie sur colonne de silice (CH₂Cl₂/MeOH 95 : 5) pour donner 0,405 g du composé tricyclique attendu sous forme de poudre :
- Rrendement = 98 %.
- RMN ¹H (CDCl₃) : 2,42 (s, 3H), 2,77 (s, 3H), 6,86 (s, 1H), 7,38 (dd, 1H, J = 8 et 1.6 Hz), 7,52 (d, 1H, J = 8 Hz), 7,61 (dd, 1H, J = 5,2 et 7,6 Hz), 7,74 (d, 1H, J = 1.6 Hz), 8,46 (dd, 1H, J = 7,6 et 5,2 Hz), 9,02 (dd, 1H, J = 2 et 5,2 Hz), 11,18 (s, 1H).

### A-2 - Synthèse de la 6-(2-acétyl-4-chloro-phénylamino)-quinoline-5,8-dione (Intermédiaire A₂)

Préparation selon le procédé décrit dans le chapitre A-1 : quinoline-5,8-dione (0,188 g, 1,18 mmol), chlorure de cérium (0,88 g, 2,36 mmol), 5-chloro-2-aminoacétophénone (0,4 g, 3,14 mmol), éthanol (10 + 4 ml), acide acétique (25 ml). On obtient 0,3 g de poudre rouge :
- Rendement = 78 %
- RMN ¹H (CDCl₃) : 2, 65 (s, 3H), 6,84 (s; 1H), 7,52 (dd, 1H, J = 8.8 Hz), 7,57 (d, 1H, J = 8,8 Hz), 7,63 (dd, 1H, J = 8 et 4,4 Hz), 7,89 (d, 1H, J = 2,4 Hz), 8,46 (dd; 1H, J = 0,8 et 8 Hz), 9,02 (dd, 1H, J = 2 et 5,2 Hz), 11,18 (s, 1H).
- RMN ¹³C (CDCl₃) : 28,5 ; 107,36 ; 121,86 ; 126,69 ; 126,85 ; 127,49 ; 128,39 ; 132,10 ; 134,06 ; 134,75 ; 138,36 ; 143,29 ; 148,32 ; 155,22 ; 181,28 ; 182,63 ; 200,39.

### A-3 - Synthèse de la 6-(2-acétyl-4-benzylamino-phénylamino)-quinoline-5,8-dione (Intermédiaire A₃)

Préparation selon le procédé décrit dans le chapitre A-1: quinoline-5,8-dione (0,250 g, 1,57 mmol), chlorure de cérium (0,77 g, 3,14 mmol), 5-benzylamino-2 aminoacétophénone (0,603 g, 3,14 mmol), éthanol (15 + 7 ml), acide acétique (35 ml). On obtient 0,56 g de poudre rouge :
- Rendement = 91 %.
- RMN ¹H (CDCl₃) : 2,54 (s, 3H), 4,38 (s, 2H), 6,70 (s, 1H), 6,83 (dd, 1H, J = 9.6 et 3,2 Hz), 7,08 (d, 1H, J = 3.2 Hz), 7,30-7,37 (m, 5H), 7,43 (d, 1H, J = 9,6 Hz), 7,58 (dd, 1H, J = 7,6 et 4,8 Hz), 8,43 (dd, 1H, J = 7,6 et 2 Hz), 9,03 (dd, 1H, J = 2 et 4,8 Hz), 10,67 (s, 1H).

### A-4 - Synthèse de la 6-(2-acétyl-5-bromo-phénylamino)-quinoline- 5,8 dione (Intermédiaire A₄) (CRL8268)

Préparation selon le procédé décrit par F. Bracher, Liebigs Ann. Chem. 1990, 205-206.

### A-5 - Synthèse de la 6-(2-acétyl-4-diméthylamino-phénylamino)-quinoline-5,8-dione (Intermédiaire A₅)

Préparation selon le procédé décrit dans le chapitre A-1 : quinoline-5,8-dione (0,36 g, 2,26 mmol), chlorure de cérium (1,67 g, 4,49 mmol), 5-diméthylamino-2 aminoacétophénone (0,8 g, 4,49 mmol), éthanol (20 + 10 ml), acide acétique (50 ml). On obtient 1,26 g de poudre rouge :
- Rendement = 84 %.
- RMN ¹H (CDCl₃) : 2,85 (s, 3H), 3,12 (s, 6H), 6,72 (s, 1H) 6,90 (dd, 1H, J = 2.8 et 9.2 Hz), 7,15 (d, 1H, J = 2,8 Hz), 7,49 (d, 1H, J = 9,2 Hz), 7,58 (dd, 1H, J = 8Hz et 4,4 Hz), 8,43 (dd, 1H, J = 1,6 et 8 Hz), 9,00 (dd, 1H, J = 1.6 et 4,4 Hz), 10,69 (s, 1H).

### A-6 - Synthèse de la 6-(2-acétyl-4-méthoxy-phénylamino)-quinoline-5,8-dione (Intermédiaire A₆)

Préparation selon le procédé décrit dans le chapitre A-1 : quinoline-5,8-dione (3,51 g, 22,08 mmol), chlorure de cérium (16,4 g, 44,03 mmol), 5-méthoxy-2-amino acétophénone (7,29 g, 44,18 mmol), éthanol (200 + 90 ml), acide acétique (500 ml). On obtient 4,25 g de poudre rouge :
- Rendement = 60 %.
- RMN ¹H (CDCl₃) : 2,65 (s, 3H), 3,87 (s, 3H), 6,76 (s, 1H) 7,12 (dd, 1H, J = 2,8 et 8,8 Hz), 7,42 (d, 1H, J = 2,8 Hz), 7,55 (d, 1H, J= 8,8 Hz), 7,61 (dd, 1H, J = 7,6 et 4,4 Hz), 8,45 (dd, 1H, J = 1,6 et 7,6 Hz), 9,01 (dd, 1H, J = 1,6 et 4,4 Hz), 10,80 (s, 1H).

### A-7 - Synthèse de la 4,6-bis(2-acétylanilino)-quinoline-5,8-dione (Intermédiaire A₇)

Préparation selon le procédé décrit dans le chapitre A-1 : 4-chloro-quinoline-5,8-dione (3,5 g, 18 mmol), chlorure de cérium (13,5 g, 36,24 mmol), 2-aminoacétophénone (4,4 ml, 36 mmol), éthanol (160 + 70 ml), acide acétique (400 ml). On obtient 2,32 g de poudre rouge :
- Rendement = 30 %.
- RMN ¹H (CDCl₃) : 2,69 (s, 3H), 2,72 (s, 3H), 6,85 (s, 1H), 7,18 (ddd, 1H, J = 7,6 et 7,6 et 0,8 Hz), 7,28 (m, 1H), 7,30 (d, 1H, J = 6,4 Hz), 7,54-7,59 (m, 3H), 7,63 (d, 1H, J = 7,6 Hz), 7,91 (dd, 1H, J = 1,6 et 8,4 Hz), 7,94 (dd, 1H, J = 1,2 et 8,4 Hz), 8,47 (d, 1H, J = 6,4 Hz), 11,35 (s, 1H), 12,35 (s, 1H).

### A-8 - Synthèse de la 6-(2-acétyl-4-bromo-phénylamino)-4-méthoxyquinoline-5,8-dione (Intermédiaire A₈)

Préparation selon le procédé décrit dans le chapitre A-1 : 4-méthoxyquinoline-5,8-dione (1,57 g, 9,1 mmol), chlorure de cérium (3,1 g, 8,3 mmol), 5-bromo-2-amino acétophénone (Léonard, Boyd, J.Org. Chem. 1946;11, 419-423) (1,95g, 9,1 mmol), éthanol (200 ml), acide acétique (180 ml). On obtient après purification par flash-chromatographie sur colonne de silice (CH₂Cl₂/MeOH 95 : 5) 1,22 g de poudre orange :
- Rendement = 37 %.
- RMN ¹H (CDCl₃) : 3,15 (s, 3H), 4,58 (s, 3H), 7,61 (d, 1H, J = 6 Hz), 7,74 (s, 1H), 7,99 (d, 1H, J = 8,8 Hz), 8,14 (dd, 1H, J = 8,8 et 2,4 Hz), 8,51 (d, 1H, J = 2,8 Hz), 9,32 (d, 1H, J = 6 Hz), 11,68 (s, 1H).

### A-9 - Synthèse de la 2-méthoxy-6-(2-acétyl-phénylamino)-quinoline-5,8-dione (Intermédiaire A₉)

A une suspension de 2-méthoxyquinoline-5,8-dione (0,54 g, 2,8 mmol) et de chlorure de cerium (1,16 g, 4,7 mmol) dans l'éthanol (100 ml), on ajoute une solution d'o-aminoacétophénone (0,41 g, 3,1 mmol) dans l'éthanol (6 ml). Le milieu réactionnel est agité, à température ambiante, pendant 40 h. Après concentration à l'évaporateur rotatif, le brut obtenu est purifié par filtration sur silice (CHCl₃/heptane 98 : 2) pour donner le produit de condensation attendu sous forme de poudre rouge (0,35 g).
- Rendement = 38 %.
- Point de fusion = 258 °C.
- ¹H RMN (CDCl₃) : 2,67 (s, 3H) ; 4,15 (s, 3H) ; 6,79 (s, 1H) ; 6,98 (d, 1H, J = 8,8 Hz) ; 7,18 (ddd, 1H, J = 8,1, 8,4 et 1,5 Hz) ; 7,56 (dd, 1H, J = 8,4 et 1,5 Hz) ; 7,61 (ddd, 1H, J = 8,1 et 8,4 et 1,1 Hz) ; 7,94 (dd, 1H, J = 8,1 et 1,5 Hz) ; 8,31 (d, 1H, J = 8,8 Hz).
- ¹³C RMN (CDCl₃) : 28,51 ; 54,73 ; 106,02 ; 115,22 ; 120,78 ; 122,50 ; 123,11 ; 125,70 ; 132,34 ; 134,24 ; 137,18 ; 140,05 ; 143,30 ; 148,21 ; 167,75 ; 180,88 ; 183,05 ; 201,41.
- IR (CHCl₃) : 1668 ; 1644 cm⁻¹.

### A-10 - Synthèse de la 3-hydroxyméthyl-6-(2-acétylphénylamino)-quinoline-5,8-dione (Intermédiaire A₁₀)

### a) - 3-hydroxyméthyl-5,8-diméthoxyquinoline

A une solution d'éthyl-5,8-diméthoxyquinoline-3-carboxylate (180 mg, 0,689 mmol) dans 60 ml de THF, une solution de LiAlH4 1M/Et₂O (5 ml, 5 mmol) est ajoutée goutte-à-goutte et sous azote. Le mélange est agité à température ambiante pendant 15 heures puis versé dans 15 ml de NaOH 1N et 40 ml d'eau. Après extraction au CH₂Cl₂ (3 x.100 ml), puis séchage de la phase organique sur MgSO₄, l'extrait est concentré à l'évaporateur rotatif. Le brut obtenu est purifié par flash-chromatographie (CH₂Cl₂ : MeOH 95 : 5) pour donner le produit attendu sous forme de poudre marron (72 mg) :
- Rendement = 48 %.
- Point de fusion = 150 °C.
- ¹H RMN (CDCl₃) : 3,92 (s, 3H) ; 4,00 (s, 3H) ; 4,88 (s, 2H) ; 6,72 (d, 1H, J = 8,4 Hz) ; 6,88 (d, 1H, J = 8,4 Hz) ; 8,47 (d, 1H, J = 2,2 Hz) ; 8,87 (d, 1H, J = 2,2 Hz).
- ¹³C RMN (CDCl₃) : 55,76 ; 56,00 ; 63,09 ; 103,95 ; 106,70 ; 121,25 ; 128,62 ; 133,35 ; 139,80 ; 148,61 ; 149,21 ; 149,41.
- IR (CDCl₃) : 3607, 3417, 1622, 1605 cm-1.

### b) - 3-hydroxyméthylquinoline-5,8-dione

Une solution de 3-hydroxyméthyl-5,8-diméthoxyquinoline (55 mg, 0,25 mmol) et de nitrate de cérium ammonium (550 mg, 1 mmol) dans un mélange CH₃CN/H₂O (3 ml/ 1 ml) est agitée à température ambiante pendant 40 min. Après addition de 5 ml d'H₂O et de 10 ml d'une solution saturée de NaHCO₃, le milieu est extrait au CH₂Cl₂ (6 x 30 ml) et on sèche les phases organiques sur MgSO₃. Après évaporation du solvant à l'évaporateur rotatif, la quinone attendue est obtenue sous forme de poudre marron (11 mg) ;
- Rendement = 22 %.
- Point de fusion = 150 °C.
- ¹H RMN (CDCl₃) : 4,95 (s, 2H) 7,06 (d, 1H, J = 10,2 Hz) ; 7,15 (d, 1H, J = 10,2 Hz) ; 8,43 (s, 1H) ; 9,03 (s, 1H).
- ¹³C RMN (CDCl₃) : 62,03 ; 128,86 ; 132,26 ; 138,00 ; 139,11 ; 141,33 ; 146,58 ; 153,10 ; 183,12 ; 184,54.
- IR (CHCl₃) : 3413 ; 1680 ; 1596 cm-1.

### c) - Synthèse de la 3-hydroxyméthyl-6-(2-acétylphénylamino)-quinoline-5,8-dione (Intermédiaire A₁₀)

A une suspension de 3-hydroxyméthylquinoline-5,8-dione (0,22 g, 1,16 mmol) et de chlorure de cérium (0.6 g, 2,43 mmoll) dans l'éthanol (40 ml), est ajoutée une solution de 2-aminoacétophénone (0,18 g, 1,33 mmol). Le milieu réactionnel est agité à température ambiante dans l'obscurité pendant 3 h. Après concentration à l'évaporateur rotatif, le brut obtenu est purifié par filtration sur silice (CH₂Cl₂/MeOH 98 : 2) pour donner le produit de condensation attendu sous forme de poudre violette (0,16 g) :
- Rendement = 42 %.
- Point de fusion = 258 °C.
- ¹H RMN (DMSO-d₆) : 2,67 (s, 3H) ; 4,73 (d, 2H, J = 5,5 Hz) ; 5,67 (t, 1H, J = 5,5 Hz) ; 6,64 (s, 1H) ; 7,30 (m, 1H) ; 7,71 (m, 2H) ; 8,12 (d, 1H, J = 8,0 Hz) ; 8,35 (d, 1H, J = 2,0 Hz) ; 8,93 (d, 1H, J = 2,0 Hz) ; 11,02 (s, 1H).
- ¹³C RMN (CDCl₃) : 28,81 ; 60,08 ; 106,52 ; 120,96 ; 123,44 ; 126,14 ; 127,23 ; 131.52 ; 132.69 ; 134,43 ; 138,91 ; 141,75 ; 143,55 ; 146,62 ; 152,81 ; 181,62 ; 181,84 ; 202,02.
- IR (CHCl₃) : 3440, 1690, 1661, 1640 cm-1.

### B - Préparation des produits intermédiaires de formule III (Schéma II)

### B-1 - Synthèse de la 9,11-diméthyl-1,6-diaza-naphtacène-5,12-dione (intermédiaire B₁)(CRL 8324)

A une solution de tricycle intermédiaire A₁- (0,4 g, 1,3 mmol) dans 12 ml d'acide acétique, on ajoute lentement 1,9 ml d'acide sulfurique en solution dans 9,6 ml d'acide acétique. Le milieu réactionnel est porté à reflux 30 minutes, puis versé, après refroidissement, dans un bécher contenant de la glace pilée. On neutralise par NH₄OH puis on extrait 4 fois au dichlorométhane. Les phases organiques sont séchées sur MgSO₄ puis évaporées. Le brut obtenu est purifié par flash-chromatographie sur colonne de silice (CH₂Cl₂/MeOH 95 : 5) pour donner 0,325 g de composé tétracycle attendu.
- Rendement = 86 %.
- RMN ¹H (CDCl₃) : 2,64 (s, 3H), 3,29 (s, 3H), 7,74 (dd, 1H, J = 7,6 et 4,8 Hz), 7,75 (dd, 1H, J = 8,4 et 1,6 Hz), 8,12 (dd, J = 1,6 Hz), 8,33 (d, 1H, J = 8,4), 8,71 (dd, 1H, J = 2 et 7,6 Hz), 9,13 (dd, 1H, J = 2 et 4,8 Hz).

### B-2 - Synthèse de la 9-chloro-11-méthyl-1,6-diaza-naphtacène-5,12-dione (Intermédiaire B₂)

Préparation selon le procédé décrit dans le chapitre B-1 : tricycle intermédiaire A₂ (0,289 g, 0,88 mmol), acide sulfurique (1,3 ml), acide acétique (8 + 6,5 ml). Après purification par flash-chromatographie (CH₂Cl₂/MeOH 95 : 5), on obtient 0,26 g de tétracycle :
- Rendement : 95 %.
- RMN ¹H (CDCl₃) : 3,25 (s, 3H), 7,76 (dd, 1H, J = 8 et 4,8 Hz), 7,85 (dd, 1H, J = 8,8 et 2 Hz), 8,33 (dd, 1H, J = 2 Hz), 8,38 (d, 1H, J = 8,8), 8,71 (dd, 1H, J = 1.6 et 8 Hz), 9,15 (dd, 1H, J = 1,6 et 4,8 Hz).

### B-3 - Synthèse de la 9-benzylamino-11-méthyl-1,6-diaza-naphtacène-5,12-dione (Intermédiaire B₃)

Préparation selon le procédé décrit dans le chapitre B-1 : intermédiaire A₃ (4 g, 10 mmol), acide sulfurique (15,1 ml), acide acétique (92 + 75 ml). Après traitement, on obtient 3,58 g de tétracycle.
- Rendement = 98 %.
- RMN ¹H (CDCl₃) : 3,09 (s, 3H), 4,52 (d, 2H), 4,86 (t, 1H), 7,06 (d, 1H, J = 2.8 Hz), 7,29 (dd, 1H, J = 9,2 et 2,8 Hz), 7,3-7,43 (m, 5H), 7,71 (dd, 1H, J = 4,8 et 8 Hz), 8,20 (d, 1H, J = 9,8 Hz), 8,69 (dd,1H, J = 1,6 et 8 Hz), 9,09 (dd, 1H, J = 1,6 et 4,8 Hz).

### B-4 - Synthèse de la 8-bromo-11-méthyl-1,6-diaza-naphtacène-5,12-dione (Intermédiaire B₄)

Préparation selon le procédé décrit par F. Bracher, Liebigs Ann. Chem. 1990, 205-206.

### B-5 - Synthèse de la 9-diméthylamino-11-méthyl-1,6-diaza-naphtacène-5,12-dione (intermédiaire B₅)

Préparation selon le procédé décrit dans le chapitre B-1 : tricycle intermédiaire A₅ (0,76 g, 2,27 mmol), acide sulfurique (3,5 ml), acide acétique (20 + 18 ml). Après traitement on obtient 0,67 g de tétracycle.
- Rendement = 93 %.
- RMN ¹H (CDCl₃) : 3,17 (s, 3H), 3,21 (s, 6H), 7,04 (d, 1H, J = 3,2 Hz), 7,51 (dd, 1H, J = 3,2 et 9,2 Hz), 7,71 (dd, 1H, J = 8 et 4,4 Hz), 8,26 (d, 1H, J = 9,2 Hz), 8,7 (dd, 1H, J = 1,6 et 8 Hz) 9,09 (dd, 1H, J = 1,6 et 4,4 Hz).

### B-6 - Synthèse de la 9-méthoxy-11-méthyl-1,6-diaza-naphtacène-5,12-dione (Intermédiaire B₆)

Préparation selon le procédé décrit dans le chapitre B-1 : tricycle intermédiaire A₆ (4,25 g, 13,18 mmol), acide sulfurique (20 ml), acide acétique (110 + 100 ml). Le produit obtenu par flash-chromatographie (CH₂Cl₂/MeOH 100 : 3) est lavé à l'éther éthylique pour donner 2,9 g de tétracycle.
- Rendement = 72 %.
- RMN ¹H (CDCl₃) : 3,25 (s, 3H), 4,02 (s, 3H), 7,49 (d, 1H, J = 3,3 Hz), 7,56 (dd, 1H, J = 3,3 et 9,3 Hz), 7,74 (dd, 1H, J = 8,3 et 4,3 Hz), 8,34 (d, 1H, J = 9,3 Hz), 8,71 (dd, 1H, J = 2,5 et 8,3 Hz) 9,12 (dd, 1H, J = 2,5 et 4,3 Hz).

### B-7 - Synthèse de la 4-(2-acétylanilino)-11-méthyl-1,6-diaza-naphtacène-5,12-dione(Intermédiaire B₇) (CRL 8332)

Préparation selon le procédé décrit dans le chapitre B-1 : tricycle intermédiaire A₇ (1 g, 2,35 mmol), acide sulfurique (3,5 ml), acide acétique (18 ml). Le produit obtenu par flash-chromatographie (CH₂Cl₂/MeCH 100 : 3) est lavé à l'éther éthylique pour donner 0,6 g de tétracycle sous forme de poudre orange.
- Rendement = 63 %.
- RMN ¹H (CDCl₃) : 2,59 (s, 3H), 3,25 (s, 3H), 7,29 (ddd, 1H, J = 7,2 et 7,2 et 1,2 Hz), 7,37 (d, 1H, J = 6 Hz), 7,54 (ddd, 1H, J = 6,8 et 6,8 et 1,6 Hz), 7,59 (d, 1H, J = 6,8 Hz), 7,74 (dd, 1H, J = 7,2 et 1,2 Hz), 7,76 (dd, 1H, J = 6,8 et 1,6 Hz), 7,87-7,918 (m, 2H), 8,34 (d, 1H, J = 8,4 Hz), 8,43 (d, 1H, J = 8,4 Hz), 8,54 (d, 1H, 6 Hz), 12,5 (s, 1H).

### B-8 - Synthèse de la 4-méthoxy-9-bromo-11-méthyl-1,6-diaza-naphtacène-5,12-dione (Intermédiaire B₈)

Préparation selon le procédé décrit dans le chapitre B-1 : tricycle intermédiaire A₈ (1,22 g, 3,04 mmol), acide sulfurique (4,5 ml), acide acétique (27 + 23 ml). Le produit obtenu par flash-chromatographie (CH₂Cl₂/MeOH 100 : 3) est lavé à l'éther éthylique pour donner 0,76 g de tétracycle sous forme de poudre jaune.
- Rendement = 65 %.
- RMN ¹H (CDCl₃) : 3,21 (s, 3H), 4,10 (s, 3H), 7,18 (d, 1H, J =6 Hz), 7,96 (dd, 1H, J = 8,8 et 2 Hz), 8,27 (d, 1H, J = 8,8 Hz), 8.47 (d, 1H, J = 2 Hz), 8,89 (d, 1H, J = 6 Hz).

### B-9 - Synthèse de 2-méthoxy-11-méthyl-1,6-diaza-naphtacène-5,12-dione, (Intermédiaire B₉)

Une solution de 2-méthoxy-6-(2-acétyl-phényiamino)-quinoline-5,8-dione (0,34 g, 1,1 mmol) dans un mélange acide acétique/acide sulfurique (25 mol/2,7 ml) est chauffée à 90 °C pendant 45 min. Après refroidissement le milieu réactionnel est versé dans un mélange eau/glace (200 ml) puis alcalinisé à pH 8 par du K₂CO₃ et extrait au CHCl₃ (3 x 200 ml). Les phases organiques sont séchées sur MgSO₄ puis concentrées à l'évaporateur rotatif. Le brut obtenu est purifié par filtration sur silice (CHCl₃) pour donner le tétracycle attendu sous forme de poudre beige (0,23 g) :
- Rendement = 71 %.
- Point de fusion = 260 °C.
- ¹H RMN (CDCl₃) : 3,32 (s, 3H) ; 4,23 (s, 3H) ; 7,14 (d, 1H, J = 8,8 Hz) ; 7,79 (ddd, 1H, J = 8,6, 8,4 et 1,2 Hz) ; 7,91 (ddd, 1H, J = 8,4, 8,6 et 1,2 Hz) ; 8,38 (dd, 1H, J = 8,6 et 1,2 Hz) ; 8,46 (dd, 1H, J = 8,4 et 1,2 Hz) ; 8,58 (d, 1H, J = 8,8 Hz).
- ¹³C RMN (CDCl₃) : 16,63 ; 54,76 ; 117,29 ; 125,29 ; 125,50 ; 125,64 ; 129,62 ; 129,75 ; 132,37 ; 132,57 ; 138,12 ; 147,73 ; 148,63 ; 149,69 ; 152,28 ; 167,77 ; 181,10 ; 183,55.
- IR (CHCl₃) : 1683 ; 1599 cm⁻¹.

### B-10- Synthèse 3-acétoxyméthyl-11-méthyl-1,6-diazanaphtacène-5,12-dione (Intermédiaire B₁₀)

Une solution de 3-hydroxyméthyl-6-(2-acétylphénylamino)-quinoline-5,8-dione *(Intermédiaire A*_{*10*}), (0,248 g, 0,77 mmol) dans un mélange acide acétique/acide sulfurique (16 ml/1,3 ml) est chauffée à 90 °C pendant 2h30. Après refroidissement le milieu réactionnel est versé dans un mélange eau/glace (15 ml) puis alcalinisé à pH 9 au Na₂CO₃. Le milieu est ensuite extrait au CH₂Cl₂ (3 x 150 ml). Les phases organiques sont séchées sur MgSO4 puis concentrées à l'évaporateur rotatif. Le brut obtenu est purifié par filtration sur silice (CH₂Cl₂ / MeOH 98 : 2) pour donner le composé attendu sous forme de . poudre marron (0,21 g).
- Rendement = 85%.
- Point de fusion = 210 °C.
- ¹H RMN (CDCl₃) : 2,18 (s, 3H) ;3,30 (s, 3H) ; 5,31 (s, 2H) ; 7,78 (ddd, 1H, J = 1,1, 6,8 et 8,1 Hz) ; 7,92 (ddd, 1H, J = 1,1, 6,8 et 8,1 Hz) ; 8,37 (dd, 1H, J = 8,1 et 1,1 Hz) ; 8,43 (dd, 1H, J = 8,1 et 1,1 Hz) ; 8,66 (d; 1H, J = 2,2 Hz) ; 9,09 (d, 1H, J = 2,2 Hz).
- ¹³C RMN (CDCl₃) : 16,06 ;20.11 ; 62,06 ; 124,81 ; 124,91 ; 129,06 ; 129.18 ; 129.29 ; 131,70 ; 132,18 ; 134,06 ; 136,09 ; 146,86 ; 147,97 ; 149,01 ; 152,19 ; 154,30 ; 169,72 ; 180,96 ; 182,34.
- IR (CHCl₃) : 3420, 1746, 1692 cm-1.

### C - Préparation des produits intermédiaires de formule IIIa (Schéma II)

### 1) Synthèse de l'acide N-(2,5-diméthoxyphényl)anthranilique (composé 4)

Un mélange d'acide 2-chlorobenzoïque (9,2 g, 60 mmol), de diméthoxyaniline (10 g, 65 mmol), de cuivre (0,96 g), de Cu₂O (0,96 g) et de K₂CO₃ (10,4 g) dans 120 ml de diglyme est porté à reflux pendant une nuit. Après évaporation du solvant, le milieu réactionnel est alcalinisé à la soude 1N. De l'éther est ajouté puis le milieu filtré sur silice et la phase éthérée éliminée. La phase aqueuse est acidifiée à l'HCl concentré puis extraite à l'acétate d'éthyle. Après séchage sur MgSO₄ et évaporation du solvant à l'évaporateur rotatif, le brut obtenu est purifié par filtration sur silice (CH₂Cl₂) pour donner le produit de condensation attendu sous forme de poudre jaune (14,5 g).
- Rendement = 89 %.
- Point de fusion = 138 °C.
- RMN ¹H RMN (CDCl₃) : 3,77 (s, 3H) ; 3,85 (s, 3H) ; 6,57 (dd, 1H, J = 8,8 et 2,9 Hz) ; 6,77 (ddd, 1H, J = 1,9 et 7,5 Hz) ; 6,87 (d, 1H, J =9,2 Hz) ; 7,04 (d, 1H, J = 2,9 Hz) ; 7,3 à 7,4 (m, 2H) ; 9,35 (slarge, 1H).
- ¹³C RMN (CDCl₃) : 55,76 ; 56,45 ; 107,30 ; 107,71 ; 112,00 ; 112,26 ; 114,70 ; 1.17,53 ; 130,78 ; 132,60 ; 134,09 ; 145,98 ; 147,71 ; 153,75 ; 172,95.
- IR (CHCl₃) : 3327 ; 1685 cm⁻¹.

### 2) Synthèse de la 2-(2,5-diméthoxyphénylamino)acétophénone (composé 5)

A un mélange d'acide N-(2,5-diméthoxyphényl)anthranilique (2 g, 73 mmol) dans 14 ml de THF, sont ajoutés 16 ml de MeLi (1,4 M / Et₂O) à 0 °C et sous N₂. Après remontée de la température, le milieu est porté à reflux pendant 2 h puis 100 ml d'eau sont ajoutés et le mélange extrait à l'éther (3 x 100 ml). Après séchage sur MgSO₄, le solvant est évaporé à l'évaporateur rotatif pour donner le dérivé attendu sous forme de solide jaune (1,49 g).
- Rendement = 75 %.
- Point de fusion = 79 °C.
- ¹H RMN (CDCl₃) : 2,64 (s, 3H) ; 3,76 (s, 3H) ; 3,84 (s, 3H) ; 6,55 (dd, 1H, J = 8,8 et 2,9 Hz) ; 6,73 (dd, 1H, J = 1,4 et 7,5 Hz) ; 6,85 (d, 1H, J =8,8 Hz) ; 7,04 (d, 1H, J = 2,9 Hz) ; 7,3 à 7,4 (m, 2H) ; 7,81 (dd, 1H, J = 1,5 et 8,0 Hz) ; 10,5 (slarge, 1H).
- ¹³C RMN (CDCl₃) : 48,15 ; 55,73 ; 56,36 ; 107,10 ; 107,72 ; 112,05 ; 114,80 ; 116,84 ; 120,09 ; 130,68 ; 132,42 ; 134,35 ; 145,98 ; 146,67 ; 153,62 ; 201,00.
- IR (CHCl₃) : 3350 ; 1642 cm⁻¹.

### 3) Synthèse de la 1,4-diméthoxy-9-méthylacridine (composé 6)

Un mélange de 2-(2,5-diméthoxyphénylamino)acétophénone (1,3 g, 48 mmol) et d'acide polyphosphorique (13 g, 133 mmol) est chauffé à 100 °C pendant 1 h. Après addition de 50 ml d'eau, le mélange est neutratisé à la soude 4M puis extrait au CHCl₃ (3 x 100 ml). Après séchage sur MgSO₄ et évaporation du solvant, le brut obtenu est purifié par filtration sur silice (CH₂Cl₂) pour donner quantitativement le dérivé tricyclique attendu sous forme d'un solide orange-marron.
- Point de fusion = 136 °C.
- ¹H RMN (CDCl₃) : 3,36 (s, 3H) ; 3,96 (s, 3H) ; 4,09 (s, 3H) ; 6,68 (d, 1H, J = 8,0 Hz) ; 6,89 (d, 1H, J = 8,4 Hz) ; 7,54 (m, 1H) ; 7,73 (m, 1H) ; 8,32 (d, 1H, J = 8,4) ; 8,36 (d, 1H, J = 8,8 Hz).
- ¹³C RMN (CDCl₃) : 17,78 ; 55,66 ; 56,13 ; 102,43 ; 105,18 ; 120,25 ; 124,28 ; 125,62 ; 126,59 ; 129,44 ; 130,81 ; 142,45 ; 144,23 ; 147,21 ; 149,46 ; 151,45.
- IR (CHCl₃) : 1685 ; 1661 cm⁻¹.

### 3) Synthèse de la 9-méthylacridine-1,4-dione (Composé 7)

Une solution de 1,4-diméthoxy-9-méthylacridine (20 mg, 0,079 mmol) et de nitrate de cérium ammonium (196 mg, 0,357 mmol) dans un mélange CH₂Cl₂/H₂O (0,5 ml / 0,25 ml) est agitée à 0 °C pendant 20 min. Après addition de 1,4 ml de H₂O et de 0,4 ml d'une solution saturée de NaHCO₃, le milieu est laissé sous agitation, puis extrait au CH₂Cl₂ (3 x 3ml). Les phases organiques sont séchées sur MgSO₄. Après évaporation du solvant à l'évaporateur rotatif, la quinone attendue est obtenue sous forme de poudre marron (15 mg).
- Rendement = 90 %.
- Point de fusion > 260 °C.
- ¹H RMN (CDCl₃) : 3,22 (s, 3H) 7.09 (d, 1H, J = 10,3 Hz) ; 7,18 (d, 1H, J = 10,3 Hz) ; 7,78 (dd, 1H, J = 8,5 et 8,5 Hz) ; 7,91 (dd, 1H, 8,5 et 8,5 Hz) ; 8,32 (d, 1H, J = 8,5) ; 8.43 (d, 1H, J = 8,5 Hz).
- ¹³C RMN (CDCl₃) 15,87 ; 124,40 ; 125,41 ; 126,30 ; 129,61 ; 132,32 ; 132,52 ; 137,88 ; 141,61 ; 147,05 ; 148,23 ; 151,23 ; 183,43 ; 186,69.
- IR (CHCl₃) : 1701; 1661 cm⁻¹.

### D - Préparation des produits intermédiaires de formule IVa (Schéma II)

### D-1 - Synthèse de la 6-méthyl-1,11-diaza-naphtacène-5,12-dione (Intermédiaire D₁)

Une solution de 9-méthylacridine-1,4-dione (200 mg, 0,896 mmol), d'acroleine-N,N-diméthylhydrazone (96 mg, 0,984 mmol) et d'anhydride acétique (1 ml) dans 20 ml de CH₂Cl₂ est agitée sous N₂, à température ambiante pendant 30 min. Après concentration du solvant, le milieu est purifié par filtration sur silice (CH₂Cl₂) pour récupérer le produit d'addition non complètement aromatique. Une suspension de ce composé et de Pd/C 10 % (20 mg) dans 4 ml de toluène est chauffée à reflux pendant 30 min. Après concentration, le brut obtenu est purifié par flash-chromatographie sur silice (CH₂Cl₂/MeOH 98 : 2) pour donner le tétracycle attendu sous forme de poudre beige (23 mg),
- Rendement = 13 %.
- ¹H RMN (CDCl₃) : 3,32 (s, 3H) 7,78-7,83 (m, 2H) ; 7,95 (ddd, 1H, J = 8,4, 7,7 et 1,5 Hz) ; 8,39 (dd, 1H, J = 8,8 et 1,5 Hz) ; 8,51 (dd, 1H, J = 7,7 et 1,5 Hz) ; 8,68 (dd, 1H, J = 8,1 et 1,9 Hz) ; 9,16 (dd, 1H, J = 4,8 et 1,9 Hz).
- ¹³C RMN (CDCl₃) 16,67 ; 124,59 ; 125,44 ; 128,39 ; 129,76 ; 129,89 ; 132,25 ; 132,54 ; 132,88 ; 135,93 ; 148,00 ; 148,59 ; 148,73 ; 152,48 ; 155,31 ; 180,81 ; 184,37.
- IR (CHCl₃) : 1703 ; 1663 cm⁻¹.

### D-2 - Synthèse de la 3-méthoxy-6-méthyl-1,11-diazanaphtacène-5,12-dione (Intermédiaire D₂)

La 3-méthoxy-6-méthyl-1,11-diazanaphtacène-5,12-dione est préparée selon la procédure décrite dans D-1, à partir d'une solution de 9-méthylacridine-1,4-dione (composé 7) (200 mg, 0,896 mmol), de 2-méthoxy-2-propénal-diméthylhydrazone (126 mg, 0,984 mmol) et d'anhydride acétique (1 ml) dans 20 ml de CH₂Cl₂.

### EXEMPLE 1

### 5-méthyl-9H-quino [4,3,2-de][1,10] phénanthrolin-9-one (CRL 8323)

On porte à reflux pendant 1 heure une solution de tétracycle intermiédiaire B₁ (1 g, 3,47 mmol) et de diméthylformamide diéthyl acétal (2 ml, 10,41 mmol) dans 7 ml de DMF. Après évaporation à sec, on ajoute du chlorure d'ammomium (2,77 g, 52 mmol) et 50 ml d'éthanol. Le milieu réactionnel est à nouveau porté à reflux 30 minutes. Après évaporation du solvant, le brut est repris à l'eau et extrait 4 fois au dichlorométhane. Les phases organiques sont séchées sur MgSO₄ puis évaporées. Après recristatlisation dans 125 ml de méthanol, on obtient 0,7 g du composé CRL 8323 attendu sous forme d'un solide jaune moutarde.
- Rendement = 67 %.
- Point de fusion = 200° C.
- RMN ¹H (CDCl₃) : 2,69 (s, 3H), 7,65 (dd, 1H, J = 8 et 4,8 Hz), 7,81 (dd, 1H, J = 8 et 1,2 Hz), 8,44 (d, 1H, J = 1,2 Hz), 8,49 (d, 1H, J = 8 Hz), 8,50 (d, 1H, J = 5,6 Hz), 8,78 (dd, 1H, J = 2 et 8 Hz), 9,15 (dd, 1H, J = 4,8 et 2 Hz), 9,24 (d, 1H, J = 5,6 Hz).
- RMN ¹³C (CDCl₃) : 22,06 ; 116,54 ; 117,87 ; 122,15 ; 123,12 ; 125,24 ; 128,74 ; 132,58 ; 133,47 ; 136,25 ; 137,19 ; 141,63 ; 143,88 ; 144,79 ; 149,16 ; 149,31 ; 152,09 ; 155,15 ; 181,53.
- SM (m/z) : 297 (17,6); 296 (34,3); 268 (25,4); 149 (50,3).

### EXEMPLE 2

### 5-chloro-9H-quino [4,3,2-de] [1,10] phénantrolin-9-one (CRL 8301)

Préparation selon le procédé décrit dans l'exemple 1 à partir du tétracycle intermédiaire B₂ (0,25 g, 0,81 mmol) et le diméthylformamide diéthyl acétal (1,5 ml, 8,75 mmol) dans le DMF (4,5 ml). Chlorure d'ammomium (2,95 g, 55 mmol), éthanol (50 ml). Après purification par flash-chromatographie (CH₂Cl₂/MeOH 98 : 2), on obtient 60 mg du composé CRL 8301 attendu sous forme d'un solide jaune.
- Rendement = 23 %.
- Point de fusion = 200° C.
- RMN ¹H (CDCl₃) : 7,68 (dd, 1H, J = 8,4 et 4,8 Hz), 7,94 (dd, 1H, J= 8,8 et 2 Hz), 8,46 (d, 1H, J = 5,6 Hz), 8,55 (d, 1H, J = 8,8 Hz), 8,63 (d, 1 H, J = 2 Hz), 8,79 (dd, 1H, J = 2 et 8,4 Hz), 9,18 (dd, 1H, J = 4,8 et 2 Hz), 9,30 (d, 1H, J = 5,6 Hz).
- RMN ¹³C (CDCl₃) : 117,07 ; 118,46 ; 122,98 ; 124,82 ; 126,12 ; 129,34 ; 133,02 ; 134,81 ; 137,00 ; 137,42 ; 137,79 ; 144,45 ; 146,35 ; 150,24 ; 150,45 ; 152,55 ; 156,02 ; 181,9.
- SM (m/z) : 319 (43); 317 (100); 291 (14,5); 290 (18); 289 (100).

### EXEMPLE 3

### 5-(benzylamino)-9H-quino[4,3,2-de][1,10] phénanthrolin-9-one (CRL 8241)

Préparation selon le procédé décrit dans l'exemple 1 à partir du tétracycle intermédiaire B₃ (3,58 g, 9,45 mmol) et le diméthylformamide diéthyl acétal (5,7 ml, 33,26 mmol) dans du DMF (19 ml). Chlorure d'ammomium (2,95 g, 55 mmol), éthanol (50 ml). Après purification par flash-chromatographie (CH₂Cl₂/MeOH 96 : 4), on obtient 2 g du composé CRL 8241 attendu sous forme de poudre lie-de-vin.
- Rendement = 55 %.
- Point de fusion = 219° C.
- RMN ¹H (CDCl₃) : 4,61 (d, 2H), 5,10 (t, 1H), 7,31 (dd, 1H, J = 8,8 Hz, J = 2.4 Hz), 7,452-7,327 (m, 5H), 7,55 (d, 1H, J = 2,4 Hz), 7,63 (dd, 1H, J = 4,4 Hz, J = 8,4 Hz), 8,29 (d, 1H, J=. 5,2 Hz), 8,36 (d, 1H, J = 8,8 Hz), 8,79 (dd, 1H, J = 1,2 Hz, J = 8,4 Hz), 9,13 (dd, 1H, J = 4,4 et 1,2 Hz), 9,14 (d, 1H, J = 5,2 Hz).
- SM (m/z) : 388 (7); 387 (100); 386 (85); 385 (25); 369 (99); 368 (44).

### EXEMPLE 4

### 5-(diméthylamino)-9H-quino[4,3,2-de][1,10]phénanthrolin-9-one (CRL 8325)

Préparation selon le procédé décrit dans l'exemple 1 à partir du tétracycle intermédiaire B₅ (0,25 g, 0,79 mmol) et le diméthylformamide diéthyl acétal (0,5 ml, 2,98 mmol) dans le DMF (5 ml). Chlorure d'ammomium (1 g, 18,7 mmol), éthanol (16 ml). Après purification par flash-chromatographie (CH₂Cl₂/MeOH 100 : 5), on obtient 170 mg du composé CRL 8325 attendu sous forme d'une poudre violette.
- Rendement = 66 %.
- Point de fusion > 260°C.
- RMN ¹H (CDCl₃) : 3,25 (s, 6H), 7,45 (dd, 1H, J = 9,2 Hz, J = 3Hz), 7,57 (d, 1H, J = 3 Hz), 7,63 (dd, 1H, J = 4,4 et 8Hz), 8,41 (d, 1H, J = 9,2 Hz), 8,43 (d, 1H, J = 5,6 Hz), 8,81 (dd, 1H, J = 2 et 7,6 Hz), 9,13 (dd, 1H, J = 4,4 et 2 Hz), 9,17 (d, 1H, J = 5,6 Hz).
- RMN ¹³C (CDCl₃) : 40,45 ; 100,84 ; 116,81 ; 118,69 ; 118,99 ; 125,19 ; 126,10 ; 129,46 ; 134,62 ; 136,03 ; 136,30 ; 139,00 ; 140,69 ; 148,16 ; 149,15 ; 151,53 ; 152,47 ; 154,83 ; 181,65.
- SM(m/z) : 326 (34,5); 325 (100); 324 (100); 254 (15,5); 253 (13,4).

### EXEMPLE 5

### 5-méthoxy-9H-quino[4,3,2-de][1,10]phénanthrolin-9-one (CRL 8297)

Préparation selon le procédé décrit dans l'exemple 1 à partir du tétracycle intermédiaire B₆ (2 g, 6,57 mmol) et le diméthylformamide diéthyl acétal (4 ml, 23,34 mmol) dans le DMF (14 ml). Chlorure d'ammomium (8 g, 149,5 mmol), éthanol (130 ml). Après purification par flash-chromatographie (CH₂Cl₂/MeOH 100 : 5), on obtient 170 mg du composé CRL 8297, attendu, sous forme d'un solide verdâtre.
- Rendement = 66 %.
- Point de fusion > 260 °C.
- RMN ¹H (CDCl₃) : 4,10 (s, 3H), 7,62 (dd, 1H, J = 9,2 Hz, J = 2,4Hz), 7,66 (dd, 1H, J = 4,4 et 8 Hz), 7,96 (d, 1H, J = 2,4 Hz), 8,48 (d, 1H, J = 2,4 Hz), 8,54 (d, 1H, J = 9,2 Hz), 8,80 (dd, 1H, J = 2,4 et 8 Hz), 9,16 (dd, 1H, J = 4,4 et 2,4 Hz), 9,25 (d, 1H, J = 5,2 Hz).
- RMN ¹³C (CDCl₃) : 30,93 ; 116,86 ; 118,41 ; 122,44 ; 125,58 ; 129,25 ; 134,96 ; 136,55 ; 137,13 ; 141,52 ; 143,67 , 149,11 ; 149,77 ; 152,37 ; 155,38 ; 161,71 ; 181,93 ; 207,00.
- SM (m/z) : 313 (26); 312 (100); 285 (2); 284 (15); 269 (15); 242 (32.5).

### EXEMPLE 8

### 5-bromo-9H-quino[4,3,2-de] [1,10]phénantrolin-9-one (CRL 8248)

A une solution d'ascididémine (0,5 g, 1,77 mmol) dans 20 ml d'acide acétique, on ajoute goutte à goutte une solution de brome (0,2 ml, 3,88 mmol) dans 5 ml d'acide acétique. Le milieu réactionnel est porté à reflux (réfrigérant bouché) 24 heures. Après refroidissement, on neutralise par une solution saturée de NaHCO₃ et on extrait 4 fois au CH₂Cl₂. Les phases organiques sont séchées sur MgSO₄ puis évaporées. Le brut obtenu est purifié par flash-chromatographie sur colonne de silice (CH₂Cl₂/MeOH 96 : 4) pour donner 0,548 g du composé CRL 8248 attendu sous forme d'un solide jaune.
- Rendement = 86 %
- Point de fusion = 208 °C
- RMN ¹H (CDCl₃) : 7,68 (dd, 1H, J = 4,4 et 8 Hz), 8,09 (dd, 1H, J = 8,8 Hz, J = 2 Hz), 8,48 (d, 1H, J = 8,8 Hz), 8,49 (d, 1H, J = 6Hz), 8,79 (dd, 1H, J = 2 et 8 Hz), 8,82 (d, 1H, J = 2 Hz), 9,18 (dd, 1H, J = 2 Hz, J = 4,4 Hz), 9,30 (d, 1H, J = 6 Hz).
- RMN ¹³C (CDCl₃) : 116,76 ; 117,04 ; 118,26 ; 124,76 ; 125,81 ; 125,93 ; 129,05 ; 134,52 ; 135,43 ; 136.72 ; 137,01 ; 144,41 ; 146,24 ; 149,93 ; 150,12 ; 152,27 ; 155,67 ; 181,69.
- SM (m/z) : 363 (99); 362 (83); 361 (100); 360 (27); 255 (9); 254 (51).

### EXEMPLE 9

### 5-amino-9H-quino[4,3,2-de][1,10]phénanthtolin-9-one (CRL 8347)

A une solution d'ascididémine bromée : CRL 8248 (2,3 g, 6,33 mmol) dans 460 ml de DMF, on ajoute l'azidure de sodium (2,34 g, 36,1 mmol). Le milieu réactionnel est porté à reflux pendant 4 heures. Après refroidissement, on évapore à sec et on reprend le solide obtenu à l'eau. On extrait 4 fois au CH₂Cl₂. Après séchage sur MgSO4 et évaporation du solvant, le brut est purifié par flash chromatographie sur colonne de silice (HCCl₃/MeOH 90 : 10) pour donner 115 mg du composé CRL 8347 attendu sous forme d'une poudre noire.
- Rendement = 6 %
- point de fusion > 260 °C
- RMN ¹H (CDCl₃) : 7,43 (dd, 1H, J = 8,8 et 2,4 Hz), 7,74 (dd, 1H, J = 4,8 et 8 Hz), 7,81 (d, 1H, J = 2,4 Hz), 8,48 (d, 1H, J = 6Hz), 8,50 (d, 1H, J = 8,8 Hz); 8,90 (dd, 1H, J = 2 et 8 Hz), 9,25 (dd, 1H, J = 2 et 4,8 Hz), 9,29 (d, 1H, J = 6 Hz).
- RMN ¹³C (DMSO) : 102,26 ; 117,13 ; 118,54 ; 121,62 ; 123,20 ; 125,34 ; 126,11 ; 129,18 ; 133,80 ; 134,83 ; 135,47 ; 138,42 ; 147,65 ; 148,29 ; 151,63 ; 152,39 ; 154,32 ; 180,35.
- SM (m/z) : 298 (32); 297 (100); 269 (4); 268 (0,5).

### EXEMPLE 17

### 5-bromo-10-méthoxy-9H-quino[4,3,2-de][1,10]phénanthrolin-9-one (CRL 8389)

Préparation selon le procédé décrit dans l'exemple 1 à partir du tétracycle intermédiaire B₈ (0,74 g, 1,93 mmol) et de diméthylformamide diéthyl acétal (1,3 ml, 7,24 mmol) dans 15 ml de DMF. Chlorure d'ammomium (1,96 g, 36,4 mmol), éthanol (200 ml). Après purification par flash-chromatographie (CH₂Cl₂/MeOH 95: 5), on obtient 210 mg du composé CRL 8389 attendu sous forme de poudre orange.
- Rendement = 42 %
- Point de fusion > 260° C
- RMN ¹H (CDCl₃) : 4,14 (s, 3H), 7,14 (d, 1H, J = 5,6 Hz), 8,05 (dd, 1H, J = 2 et 8,8 Hz), 8,43 (d, 1H, J = 6 Hz), 8,44 (d, 1H, J = 8,8 Hz), 8,76 (d, 1H, J = 2 Hz), 8,95 (d, 1H, J = 6 Hz), 9,27 (d, 1H, J = 5,6 Hz).
- RMN ¹³C (CDCl₃) : 57,12 ; 109,52 ; 117,00 ; 117,76 ; 119,46 ; 121,58 ; 124,81 ; 125,52 ; 134,72 ; 135,49 ; 137,00 ; 144,85 ; 146,51 ; 147,24 ; 147,92 ; 150,43 ; 156,21 ; 167,98 ; 180,57.
- SM (m/z) : 393 (100); 392 (61.7); 391 (99,2); 390 (17,4) ; 362 (9,2) ; 333 ( 9,8) ; 254 (34,5).

### EXEMPLE 18

### 5-amino-10-méthoxy-9H-quino[4,3,2de][1,10]phénanthrolin-9-one

Une solution de composé CRL 8389 (0,5 g, 1,3 mmol) et de NaN₃ (0,5 g, 7,7 mmol) dans 20 ml de DMF est chauffée à 90 °C pendant 10 h. Après concentration, le résidu est repris par KOH 1N (35 ml), puis extrait au CH₂Cl₂/MeOH 95 : 5 (4 x 200 ml). Après séchage sur MgSO4 et concentration à l'évaporateur rotatif, le brut obtenu est purifié par flash-chromatographie sur silice (CH₂Cl₂/MeOH 80 : 20) pour donner du composé CRL 8389 attendu sous forme de poudre violette (65 mg).
- Rendement = 15 %.
- Point de fusion > 260 °C.
- RMN ¹H (DMSO-d₆) : 4,07 (s, 3H) ; 6,62 (s, 2H) ; 7,36 (d, 1H, J = 8,8 Hz) ; 7,41 (d, 1H, J = 5,9 Hz) ; 7,74 (s, 1H) ; 8,08 (d, 1H, J = 8,8 Hz) ; 8,48 (d, 1H, J = 5,2 Hz) ; 8,86 (d, 1H, J = 5,9 Hz) ; 9,08 (d, 1H, J = 5,2 Hz).
- IR (KBr) : 3420, 3196, 1636, 1616 cm-1.

### EXEMPLE 19

### Chlorhydrate de la 5-amino-9H-quino[4,3,2-de][1,10]phénanthrolin-9-one (CRL 8406)

Une solution de 5-amino-9*H*-quino[4,3,2-*de*][1,10]phénanthrolin-9-one (1 g, 3,35 mmol) et d'HCl concentré (0,56 ml) dans 200 ml de méthanol est agitée à température ambiante pendant 1 h. On ajoute 200 ml d'éther et après avoir laisser précipiter le sel, le milieu est filtré pour récupérer le composé CRL 8406 attendu sous forme de poudre noire (1 g).
- Rendement = 90 %).
- RMN ¹H (DMSO-d₆) : 7,44 (dd, 1H, J = 8,8 et 2,2 Hz) ; 7,81 (d, 1H, J = 2,2 Hz) ; 7,93 (dd, 1H, J = 5,6 et 5,9 Hz) ; 8,12 (d, 1H, J = 8,8 Hz) ; 8,66 (d, 1H, J = 5,6 Hz) ; 8,75 (d, 1H, J = 5,9 Hz).; 9,07 (d, 1H, J = 5,9 Hz) ; 9,14 (d, 1H, J = 5,9 Hz).
- IR (KBr) : 3404, 3287, 3170, 1681, 1676, 1649 cm-1.

### EXEMPLE 20

### Chlorhydrate de la 5-(diméthylamino)-9H-quino[4,3,2-de][1,10] phénanthrolin-9-one (CRL 8407).

Une solution de 5-(diméthylamino)-9*H*-quino[4,3,2-*de*][1,10]phénanthrolin-9-one (1 g, 3,06 mmol) et d'HCl concentré (0,3 ml) dans 120 ml de CHCl₃ est agitée à température ambiante pendant 45 min. Après addition de 350 ml d'éther, puis précipitation du sel, le milieu est filtré pour récupérer le produit attendu sous forme de poudre bleu marine (0,97 g).
- Rendement = 87 %.
- Point de fusion > 260 °C.

### EXEMPLE 21

### Chlorhydrate de la 5-(benzylamino)-9H-quino[4,3,2-de][1,10] phénanthrolin-9-one (CRL 8416)

Une solution de 5-(benzylamino)-9*H*-quino[4,3,2-*de*][1,10]phénanthrolin-9-one ASC20 (0,94 g, 2,42 mmol) et d'HCl concentré (0,2 ml) dans 40 ml de CHCl₃ est agitée à température ambiante pendant 30 min. On évapore le solvant et on ajoute 150 ml d'éther et après avoir laisser précipiter le sel, on filtre pour récupérer le du composé CRL 8416 attendu sous forme de poudre noire (0,98 g).
- Rendement = 95 %
- Point de fusion > 260 °C.

### EXEMPLE 22

### 5-(diméthylamino-2-éthyl)amino-9H-quino[4,3,2de][1,10] phénanthrolin-9-one (CRL 8419)

A un mélange du composé CRL 8347 (2,56 g, 8,59 mmol) et de diméthylformamide de diéthyl acétal (7,9 ml, 43,3 mmol), sont ajoutés 25 ml (166 mmol) d'acide trifluoroacétique à 0°C. Le milieu réactionnel est maintenu sous agitation pendant 5 minutes puis du cyanoborohydrure de sodium (8,2 g, 130 mmol) est ajouté par portions. Le milieu réactionnel est alors chauffé et maintenu à 95°C. Au bout de 18 heures, le mélange est alcalinisé, à pH 8, par une solution saturée de NaHCO₃ (environ 600 ml), puis extrait au CHCl₃/MeOH 95: 5 (3 x 800 ml). Les phases organiques sont lavées à l'eau puis séchées sur MgSO₄. Après évaporation du solvant à l'évaporateur rotatif, le brut obtenu est purifié par filtration sur alumine (CHCl₃ puis CHCl₃/MeOH 95) pour donner 1,15g du composé CRL 8419 attendu sous forme de poudré noire.
- Rendement = 36 %.
- Point de fusion : se décompose avant de fondre.
- RMN ¹H (CDCl₃) : 2,37 (s, 6H), 2,62 (t, 2H, J = 7,32 Hz), 3,70 (t, 2H, J = 7,32 Hz), 7,39 (dd, 1H, J = 9,2 et 3 Hz), 7,62 (dd, 1H, J = 8,0 et 4,5 Hz), 7,66 (d, 1H, J = 3 Hz), 8,35 (d, 1H, J = 9,2 Hz), 8,38 (d, 1H, J = 5,7 Hz), 8,79 (dd, 1H, J = 8,0 et 1,8 Hz), 9,12 (dd, 1H, J = 4,5 et 1,8 Hz), 9,15 (d, 1H, J = 5,7 Hz.
- RMN ¹³C (CDCl₃) : 45,97 ; 50,31 ; 56,40; 101,05 ; 116,81 ; 118,48 ; 118,89 ; 125,22 ; 126,30 ; 129,35 ; 134,87; 135,97 ; 136,32 ; 138,91 ; 140,55 ; 148,25; 148,98; 149,69 ; 152,23 ; 154;82 ; 181,37 .
- IR (CHCl₃) : 1663 cm⁻¹.
- SM (m/z) : 369 (100); 354 (15); 236 (37).

### EXEMPLE 23

### Chlorhydrate de la 5-(diméthylamino-2-éthyl)amino-9H-quino[4,3,2de][1,10] phénanthrolin-9-one (CRL 8418)

A 1,2g (3,25 mmol) de composé CRL 8419 en solution dans 60 ml de chloroforme, sont ajouté 265µl (3,25 mmol) d'acide chlorhydrique concentré. Le milieu réactionnel est maintenu sous agitation 2 heures à température ambiante. Le précipité formé est filtré puis lavé à l'éther. Le composé CRL 8418 (0.93g) est obtenu sous forme de poudre noire.
- Rendement = 70 %.
- RMN ¹H (DMSO d₆) : 2,67 (s, 6H), 3,09 (m, 2H), 4,01 (m, 2H), 7,67 (dm, 1H, J = 9,2 Hz), 7,80 (dd, 1H, J = 8,0 et 4,5 Hz), 7,94 (m, 1H), 8,26 (d, 1H, J = 9,2 Hz), 8,64 (d, 1H, J = 5,7 Hz), 9,09 (m, 1H), 9,12 (dd, 1H, J = 4,5 et 1,8 Hz), 9,14 (d, 1H, J = 5,7 Hz).

### EXEMPLE 24

### 5-bis(2-chloroéthyl)amino-9H-quino[4,3,2de][1,10] phénanthrolin-9-one (CRL 8422)

A une solution de 5-amino-9*H*-quino[4,3,2-*de*][1,10]phénanthrolin-9-one (1 g, 3,95 mmol) et de chloroacétaldéhyde (50 % aqueux, 2,6 ml ; 16,8 mmol) dans l'acide acétique (30 ml), sont ajoutées, par petites portions et à 0°C, 10 mmol de cyanoborohydrure de sodium NaBH₃CN (0,63 g). Le milieu réactionnel est maintenu sous agitation à 0 °C pendant 5 minutes, puis à température ambiante pendant 30 minutes. Ensuite, le milieu est alcalinisé à l'aide d'une solution saturée de carbonate acide de sodium NaHCO₃, puis extrait par un mélange CHCl₃/MeOH 95 : 5. Les phases organiques sont séchées sur sulfate de magnésium MgSO₄ et concentrées à l'évaporateur rotatif. Le brut obtenu est purifié par filtration sur silice (CHCl₃ puis CHCl₃/MeOH 99 : 1) pour donner deux composés : CRL 8422 et CRL 8423 (décrit dans l'exemple 25).

La 5-bis(chloroéthyl)amino-9H-quino[4,3,2de][1,10] phénanthrolin-9-one (CRL 8422) a été obtenue sont forme de poudre rose (0,14 g) :
- Rendement = 10%.
- Point de fusion = 220 °C.
- IR (KBr) : 1666 ; 1650 cm⁻¹.
- RMN ¹H (CDCl₃) : 3,83 (t, 4H, J = 7,0 Hz) ; 4,04 (t, 4H, J = 7,0 Hz) ; 7,47 (dd, 1 H, J = 9,5 et 2,9 Hz) ; 7,66 (dd, 1H, J = 8,0 et 4,4 Hz) ; 7,70 (d, 1 H, J = 2,9 Hz) ; 8,42 (d, 1H, J = 5,6 Hz) ; 8,50 (d, 1H, J = 9,5 Hz) ; 8,81 (dd, 1H, J = 8,0 et 1,8 Hz) ; 9,16 (dd, 1H, J = 4,4 et 1,8 Hz) ; 9,23 (d, 1 H, J = 5,6 Hz).
- RMN ¹³C (CDCl₃) : 40,16 ; 53,60 ; 101,70 ; 116,60 ; 118,37 ; 118,68 ; 125,39 ; 125,91 ; 129,25 ; 135,13 ; 136,12 ; 136,38 ; 139,42 ; 141,93 ; 148,24 ; 148,73 ; 149,34 ; 152,22 ; 155,08 ; 181,43.

### EXEMPLE 25

### 5-(2-chloroéthyl)amino-9H-quino[4,3,2de][1,10] phénanthrolin-9-one (CRL 8423)

Selon le procédé décrit dans l'exemple 24, 0,22 g de composé CRL 8423 sont obtenus sous forme de poudre violette. Les caractéristiques du composé CRL 8423 sont les suivantes :
- Rendement = 18 %.
- Point de fusion = 196°C.
- IR (KBr) 3413 ; 3275 ; 1654 ; 1617cm⁻¹.
- RMN ¹H (CDCl₃) : 3,81 (t, 2H, J = 5,5 Hz) ; 3,88 (t, 2H, J = 5,5 Hz) ; 5,01 (slarge, 1H) ; 7,34 (dd, 1H, J = 8,8 et 2,5 Hz) ; 7,60 (d, 1H, J = 2,5 Hz) ; 7,65 (dd, 1H, J = 7,5 et 4,4 Hz) ; 8,41 (d, 1H, J = 5,8 Hz) ; 8,43 (d, 1H, J = 8,8 Hz) ; 8,82 (dd, 1H, J = 7,5 et 1,5 Hz) ; 9,15 (dd, 1H, J = 4,4 et 1,5 Hz) ; 9,21 (dd, 1H, J = 5,8 Hz).
- RMN ¹³C (CDCl₃) : 42,83 ; 45,01 ; 100,76 ; 116,81 ; 118,78 ; 120,85 ; 125,38 ; 126.35 ; 129,35 ; 135,04 ; 136,04 ; 136,43 ; 140,22 ; 141,56 ; 148,49(2C); 149,41 ; 152,30 ; 155,07 ; 181,57.

### EXEMPLE 27

### 4-bromo-5-amino-9-H-quino[4,3,2-de][1,10] phénanthrolin-9-one (CRL 8478)

A une suspension de composé CRL 8347 (0,2 g, 0,67 mmol) dans l'acide acétique (8 ml), on ajoute du brome (35 µL, 0,67 mmol). Le milieu réactionnel est chauffé à 50 °C pendant 6 h. Après concentration, le milieu est alcalinisé à la soude 5N (20 ml) puis extrait avec un mélange 5 % MeOH/CHCl₃ (400 ml). Après séchage sur MgSO4 et évaporation du solvant, le composé CRL 8478 est obtenu sous forme d'une poudre violette qui est recristallisée dans un mélange CHCl₃/pentane 20 ml/15 ml (152 mg).
- Rendement = 61 %.
- Point de fusion > 260 °C.
- ¹H RMN (DMSO-d₆) : 7,07 (slarge, 2H) ; 7,61 (d, 1H, J = 8,8 Hz) ; 7,77 (dd, 1H, J = 7,7 et 4,0 Hz) ; 8,18 (d, 1H, J = 8,8 Hz) ; 8,61 (d, 1H, J = 7,7 Hz) ; 9,10 (d, 1H, J = 4,0 Hz) ; 9,14 (d, 1H, J = 5,9 Hz) ; 9,91 (d, 1H, J = 5,9 Hz).
- IR (CHCl₃) : 3501 ; 3400 ; 1673 cm⁻¹.
- MS : m/z 378 (42) ; 377 (100) ; 376 (48) ; 375 (27).

### EXEMPLE 30

### 5-bromo-9-H-quino[4,3,2-de][1,7] phénanthrolin-9-one (CRL 8839)

Le composé CRL 8839 est préparé selon la procédure décrite dans l'exemple 8 à partir de la 9-*H*-quino[4,3,2-*de*][1,7]phénanthrolin-9-one (CRL 8529) (0,5g, 1,77 mmol) ; 20 ml d'acide acétique ; solution de brome (0.2ml, 3,88 mmol/5ml d'acide acétique) ; reflux 24 heures.

### EXEMPLE 31

### 5-amino-9-H-quino[4,3,2-de][1,7] phénanthrolin-9-one (CRL 8836)

Le composé CRL 8836 est préparé selon la procédure décrite dans l'exemple 9 à partir de la 5-bromo-9-*H*-quino[4,3,2-*de*][1,7Jphénanthrolin-9-one (CRL 8839) (1,15g, 18 mmol); 250 ml de DMF ; azidure de sodium (1,2g, 1,85 mmol) ; reflux 4 heures.

### EXEMPLE 32

### 5-(diméthylamino-2-éthyl)amino-9-H-quino[4,3,2-de][1,7] phénanthrolin-9-one (CRL 8840)

Le composé CRL 8840 est préparé selon la procédure décrite dans l'exemple 22 à partir de la 5-amino-9-*H*-quino[4,3,2-*de*][1,7]phénanthrolin-9-one (CRL 8836) (1,28g, 4,3 mmol); diméthylformamide de diacétal (4 ml, 21,9 mmol); acide trifluoroacétique (12,5 ml, 83 mmol) ; cyanoborohydrure de sodium (4,1 g, 65 mmol) ; 90 °C pendant 8 heures.

### EXEMPLE 33

### 5-bis(chloroéthylamino-2-éthyl)amino-9-H-quino[4,3,2-de][1,7]phénanthrolin-9-one (CRL 8841)

Le composé CRL8841 est préparé selon la procédure décrite dans l'exemple 24 à partir de la 5-amino-9-*H*-quino[4,3,2-*de*][1,7]phénanthrolin-9-one (CRL 8836) (1g, 3,95 mmol); chloroacétaldéhyde (2,6 ml, 16,8 mmol) ; acide acétique (30 ml); cyanoborohydrure de sodium (0,63 g, 10 mmol); 30 minutes à température ambiante.

### EXEMPLE 34

### 5-(chloroéthylamino-2-éthyl)amino-9-H-quino[4,3,2-de][1,7] phénanthrolin-9-one (CRL 8842)

Le composé CRL 8842 est également obtenu lors de la procédure décrite dans l'exemple précédant à partir de la 5-amino-9-*H*-quino[4,3,2-*de*][1,7] phénanthrolin-9-one (CRL 8836).

### EXEMPLE 35

### 4-bromo-5-amino-9-H-quino[4,3,2-de][1,7] phénanthrolin-9-one (CRL 8843)

Le composé CRL 8843 est préparé selon la procédure décrite dans l'exemple 27 à partir de la 5-amino-9-*H*-quino[4,3,2-*de*][1,7] phénanthrolin-9-one (CRL 8836) (0,6g, 2,01 mmol) ; 24 ml d'acide acétique ; brome (35 µl, 0,67 mmol) ; 50°C pendant 6 heures.

Les résultats des essais pharmacologiques, présentés ci-après, mettent en évidence les propriétés cytotoxiques des composés de formules I et Ia , ainsi que les doses maximales tolérées. Ces données permettent d'apprécier l'intérêt thérapeutique des composés revendiqués.

### 1- Détermination de la dose maximale tolérée (DMT)

L'évaluation de la dose maximale tolérée a été réalisée chez des souris B6D2F1/Jico âgées de 4 à 6 semaines. Les composés ont été administrés par voie intrapéritonéale à des doses croissantes s'échelonnant de 2,5 à 160 mg/kg. La valeur de la DMT (exprimée en mg/kg) est déterminée à partir de l'observation du taux de survie des animaux sur une période de 14 jours après une administration unique du produit considéré. L'évolution pondérale des animaux est également suivie pendant cette période. Lorsque la valeur de la DMT est supérieure à 160 mg/kg, la valeur de la DMT est assimilée à 160 mg/kg par défaut.

Les résultats de l'estimation de la dose maximale tolérée (DMT) sont rassemblés dans le tableau I suivant :

**TABLEAU I**

| **Composés CRL** | **DMT (mg/kg)** |
|---|---|
| CRL 8274 (Ascididémine) | 20 |
| CRL 8269(2-bromo)eptoctinidone) | 40 |
| CRL 8323 (Exemple 1) | 20 |
| CRL 8301 (Exemple 2) | >160 |
| CRL 8241 (Exemple 3) | >160 |
| CRL 8325 (Exemple 4) | >160 |
| CRL 8297 (Exemple 5) | >160 |
| CRL 8248 (Exemple 8) | >160 |
| CRL 8347 (Exemple 9) | >160 |
| CRL 8389 (Exemple 17) | >160 |
| CRL 8406 (Exemple 19) | >160 |
| CRL 8407 (Exemple 20) | >160 |
| CRL 8416 (Exemple 21) | >160 |
| CRL 8419 (Exemple 22) | 40 |
| CRL 8418 (Exemple 23) | 40 |
| CRL 8422 (Exemple 24) | >160 |
| CRL 8423 (Exemple 25) | >160 |

La majorité des produits décrits dans la famille de l'ascididémine ou de son isomère ne présentent pas de toxicité directe (DMT > 160 mg/kg) et peuvent donc être utilisés *in vivo* à concentrations tissulaires élevées, donc à des posologies fortes.

### 2- Activité cytotoxique sur des lignées cellulaires tumorales en culture

L'influence des composés de formules I et la sur les cellules néoplasiques a été évaluée à l'aide du test colorimétrique MTT. Le principe du test MTT est basé sur la réduction mitochondriale par les cellules vivantes métaboliquement actives du produit MTT (3-(4,5-diméthylthiazol-2-yl)-2,5 diphényltétrazolium bromide) de couleur jaune en un produit de couleur bleue, le formazan. La quantité de formazan ainsi obtenue est directement proportionnelle à la quantité de cellules vivantes présentes dans le (ou les) puit(s) de culture. Cette quantité de formazan est mesurée par spectrophotométrie.

Les lignées cellulaires sont maintenues en culture monocouche à 37°C dans des boîtes de culture à bouchon fermé contenant du milieu de base MEM 25 MM HEPES (Minimum Essential Medium). Ce milieu, adapté à la croissance d'une gamme de cellules variées diploïdes ou primaires de mammifères, est ensuite additionné :
- d'une quantité de 5% de SVF (Sérum de Veau Foetal) décomplémenté à 56°C pendant 1 heure,
- de 0,6 mg/ml de L-glutamine,
- de 200 IU/ml de pénicilline,
- de 200 mg/ml de streptomycine,
- de 0,1 mg/ml de gentamicine.

Les 12 lignées cellulaires cancéreuses humaines utilisées ont été obtenues auprès de *l'American Type Culture Collection* (ATCC, Rockville, MD, USA). Ces 12 lignées cellulaires sont :
- U-373MG (code ATCC : HTB-17) et U-87MG (code ATCC : HTB-14) qui sont deux glioblastomes,
- SW1088 (code ATCC : HTB-12) qui est un astrocytome,
- A549 (code ATCC : CCL-185) et A-427 (code ATCC : HTB-53) qui sont deux cancers du poumon non-à-petites-cellules,
- HCT-15 (code ATCC : CCL-225) et LoVo (code ATCC : CCL-229) qui sont deux cancers colorectaux,
- T-47D (code ATCC : HTB-133) et MCF7 (code ATCC : HTB-22) qui sont deux cancers du sein,
- J82 (code ATCC : HTB-1) et T24 (code ATCC : HTB-4) qui sont deux cancers de la vessie,
- PC-3 (code ATCC : CRL-1435) qui est un cancer de la prostate.

Au plan expérimental : 100 µl d'une suspension cellulaire contenant 20 000 à 50 000 (selon le type cellulaire utilisé) cellules/ml de milieu de culture sont ensemencés en plaques multi-puits de 96 puits à fond plat et sont mis à incuber à 37°C, sous atmosphère comprenant 5% de CO₂ et 70% d'humidité. Au bout de 24 heures d'incubation, le milieu de culture est remplacé par 100 µl de milieu frais contenant soit les différents composés à tester à des concentrations variant de 10⁻⁵ à 10⁻¹⁰ M soit le solvant ayant servi à la mise en solution des produits à tester (condition contrôle). Après 72 heures d'incubation dans les conditions précédentes, le milieu de culture est remplacé par 100 µl d'une solution jaunâtre de MTT dissous à raison de 1 mg/ml dans du RPMI 1640. Les microplaques sont remises à incuber pendant 3 heures à 37°C puis centrifugées pendant 10 minutes à 400 g. La solution jaunâtre de MTT est éliminée et les cristaux de formazan bleu formés au niveau cellulaire sont dissous dans 100 µl de DMSO. Les microplaques sont ensuite mises sous agitation pendant 5 minutes. L'intensité de la coloration bleue résultant donc de la transformation du produit MTT jaune en formazan bleu par les cellules encore vivantes au terme de l'expérience est quantifiée par spectrophotométrie à l'aide d'un appareil de type *DYNATECH IMMUNOASSAY SYSTEM* aux longueurs d'onde de 570 nm et 630 nm correspondant respectivement aux longueurs d'ondes d'absorbance maximale du formazan et au bruit de fond. Un logiciel intégré au spectrophotomètre calcule les valeurs moyennes de densité optique ainsi que les valeurs de déviation standard (Dév. Std.) et d'erreur standard sur la moyenne (ESM).

L'activité inhibitrice de la croissance cellulaire des composés de formules I et la sur les différentes lignées cellulaires tumorales a été comparée à celle du produit naturel, l'ascididémine (CRL 8274). L'ensemble des composés présentent une activité inhibitrice significative de la prolifération cellulaire des 12 lignées tumorales humaines : U-87MG, U-373MG, SW 1088, T24, J82, HCT-15, LoVo, MCF7, T-47D, A549, A-427 et PC-3 avec une concentration inhibitrice 50 % (Cl 50) qui est comprise entre 10⁻⁶M et 10⁻¹⁰M, selon les composés et les lignées tumorales testés. A titre d'exemple, les valeurs des concentrations encadrant les Cl50 obtenues sur les différentes lignées cellulaires sont données dans le tableau II :

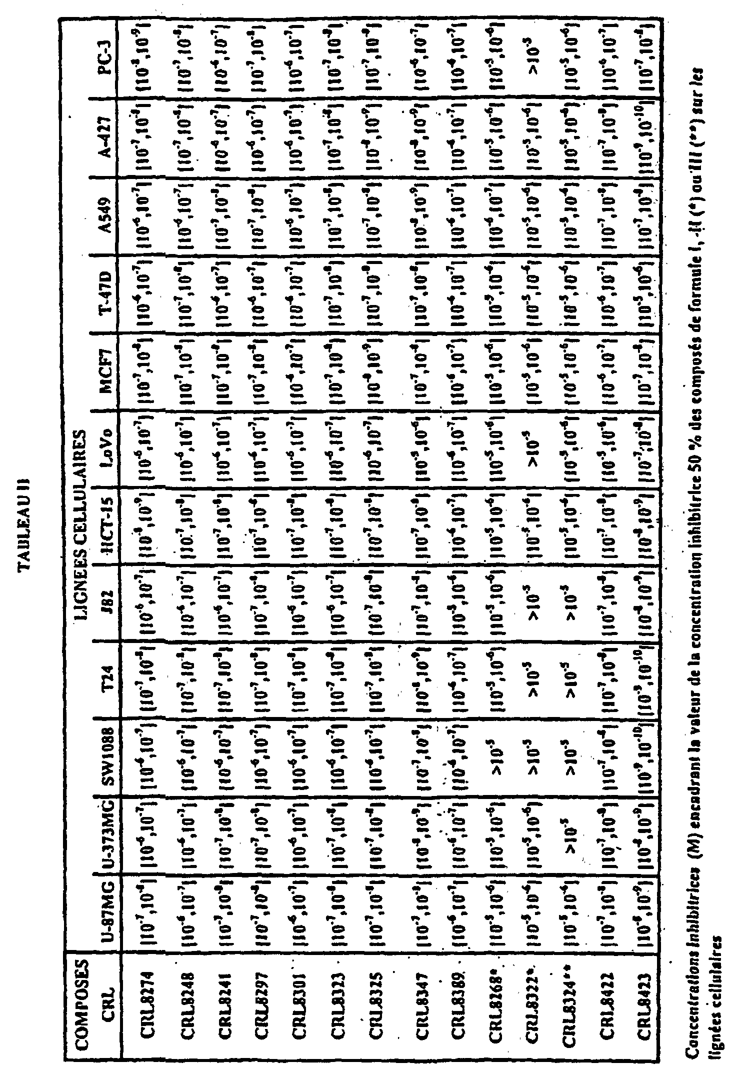

On donnera, dans le tableau III, les résultats des Cl50 (en nM) moyennes (calculées à partir de l'activité cytotoxique sur les 12 lignées tumorales étudiées) et les ratios DMT/Cl50 (ces ratios sont calculés en faisant le rapport des DMT et des Cl50 exprimées en nombres sans dimension).

**TABLEAU III**

| Composés CRL | Cl 50 (nM) | DMT/CI50 | DMT/CI50* |
|---|---|---|---|
| CRL 8274 (Ascididémine) | 100 | 0,20 | 1 |
| CRL 8269 (2-bromoleptoclinidone) | 120 | 0,33 | 2 |
| CRL 8248 (Exemple 8) | 80 | 2,00 | 10 |
| CRL 8241 (Exemple 3) | 140 | 1,14 | 6 |
| CRL 8297 (Exemple 5) | 90 | 1,78 | 9 |
| CRL 8325 (Exemple 4) | 37 | 4,32 | 22 |
| CRL 8347 (Exemple 9) | 21 | 7,62 | 38 |
| CRL 8323 (Exemple 1) | 60 | 0,33 | 2 |
| CRL 8301 (Exemple 2) | 270 | 0,59 | 3 |
| CRL 8389 (Exemple 17) | 420 | 0,38 | 2 |
| CRL 8406 (Exemple 19) | 60 | 2,67 | 13 |
| CRL 8407 (Exemple 20) | 22 | 7,27 | 36 |
| CRL 8416 (Exemple 21) | 80 | 2,00 | 10 |
| CRL 8418 (Exemple 23) | 110 | 0,37 | 2 |
| CRL 8419 (Exemple 22) | 60 | 0,67 | 3,3 |
| CRL 8422 (Exemple 24) | 100 | 1,60 | 8,3 |
| CRL 8423 (Exemple 25) | 7 | 22,86 | 114 |

| | | | |
|---|---|---|---|
| * : le ratio DMT/IC50 des différents composés a été estimé en prenant comme référence un ratio égal à 1 pour l'ascididémine. | | | |

Les composés décrits présentent, sur les modèles de lignées cellulaires tumorales des Cl 50 (nM) supérieures ou équivalentes à celle de l'ascididémine. Les doses maximales tolérées des composés décrits, considérées par défaut équivalente à 160 mg/kg, sont nettement supérieures à celle de l'ascididémine (20 mg/kg). Ces résultats suggèrent que cette nouvelle famille de composés ne présentent pas de toxicité directe. En conséquence, les ratios tolérance/activité cytotoxique des composés exemplifiés dans la présente invention, sont nettement supérieurs à celui de l'ascididémine naturelle. Ces composés peuvent donc être utilisés comme médicament anti-tumoraux, pour leurs propriétés cytotoxiques à des concentrations tissulaires plus élevées que celles induites par l'ascididémine naturelle. Ils sont donc caractérisés par une meilleure maniabilité thérapeutique.

Grâce à leurs propriétés cytotoxiques, les composés de formules I et la tels que décrits ou sous forme de sels ou solvates pharmaceutiques acceptables, peuvent être utilisés comme principes actifs de médicaments.

Les composés de formules I et la sont généralement administrés en unités de dosage établies soit par m² de surface corporelle, soit par kg de poids. Les dites unités de dosage sont de préférence formulées. dans des compositions pharmaceutiques dans lesquelles le principe actif est mélangé avec un (ou plusieurs) excipient(s) pharmaceutique(s).

Les composés de formules I et la peuvent être utilisés selon la pathologie cancéreuse du sujet à traiter à des doses comprises entre 0,05 et 350 mg/m² de surface corporelle, de préférence à des doses de 0,5 à 50 mg/m²/jour pour un traitement curatif dans sa phase aiguë en fonction du nombre de cycles de traitement de chaque cure. Pour un traitement d'entretien, on utilisera avantageusement les composés de formules I et la à des doses de 0,05 à 25 mg/m²/jour, de préférence à des doses de 0,1 à 1,5 mg/m²/jour selon le nombre de cycles de traitement de la cure. Ils pourront être associés aux médicaments anti-tumoraux utilisés dans les protocoles validés de polychimiothérapie intensive.

Dans les compositions pharmaceutiques de la présente invention pour l'administration par voie orale, intraveineuse, les principes actifs peuvent être administrés sous formes unitaires d'administration, en mélange avec des supports pharmaceutiques classiques adaptés à la thérapeutique humaine. Les formes unitaires d'administration appropriées comprennent les formes par voie orale telles que les comprimés, éventuellement sécables, ou les gélules, les implants et les formes d'administration intraveineuse. Pour une administration parentérale (perfusion intraveineuse à débit constant), on utilise des suspensions aqueuses stériles, des solutions salines isotoniques stériles ou des solutions stériles et injectables qui contiennent des agents de dispersion et/ou des agents solubilisants pharmacologiquement compatibles, par exemple le propylèneglycol, le polyéthylèneglycol, ou une β cyclodextrine.

Ainsi, pour préparer une solution aqueuse injectable par voie intraveineuse et destinée à une perfusion réalisée sur 1 à 24 h, on peut utiliser un cosolvant : un alcool tel que l'éthanol, un glycol tel que le polyéthylèneglycol ou le propylèneglycol et un tensioactif hydrophile tel que le Tween 80.

Lorsque l'on prépare une composition solide sous forme de comprimés, on peut ajouter au principe actif, micronisé ou non, un agent mouillant tel que le laurylsulfate de sodium et on mélange le tout avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α-, β- ou γ-cyclodextrine, 2-hydroxypropyl-β-cyclodextrine ou méthyl-β-cyclodextrine.

Les composés de formules I seront utilisés dans le, traitement de la plupart des tumeurs solides du fait de leurs activités cytotoxiques puissantes, en particulier pour traiter les tumeurs cérébrales, les cancers du poumon, les tumeurs de l'ovaire et du sein, les cancers de l'endomètre, les cancers colo-rectaux, les cancers de la prostate et les tumeurs testiculaires.

## Revendications

1. Composition pharmaceutique comprenant une quantité efficace d'un composé choisi parmi les composés de formules I et la pour traiter, grâce à leurs. propriétés cytotoxiques, les tumeurs cancéreuses et leurs métastases : 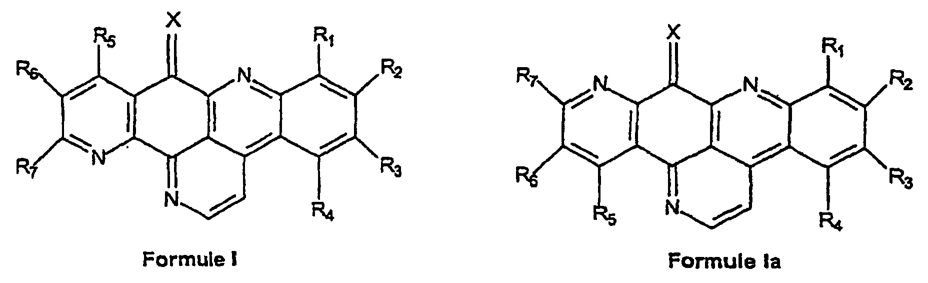 dans lesquelles :
- X est choisi parmi l'oxygène, le groupe =NH, le groupe = N-OH,
- R₁ est choisi parmi l'hydrogène, les halogènes, le groupé nitro et les groupes -NR₈R₉ dans lesquels R₈ et R₉ sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes alkyle (C₁-C₄), phénylalkyle (C₁-C₄), et les groupes -(CH₂)ₙ-Y avec Y est choisi parmi les halogènes et les groupes CN, -CH(O-Et)₂, alcoxy(C₁-C₆), -O-(CH₂)₂-N(CH₃)₂, -N(CH₃)₂ et n = 1 à 3,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
l'hydrogène, un atome d'halogène,
les groupes alkyle en C₁-C₆, hydroxyle, atcoxy en C₁-C₆, alcoxy(C₁-C₆)alkyle(C₁-C₆), alkyl(C₁-C₄) carbonyloxyalkyle(C₁-C₄), -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis, indépendamment l'un de l'autre, parmi l'hydrogène, les groupes alkyle (C₁-C₆), -phényle-CO-CH₃ et -CH₂-CH₂-N(CH₃)₂,
les groupes ―phényle-CO-CH₃ ou ―phényle-CO-CH=CH―N(CH₃)₂, morpholino, nitro, SO₃H,
les groupes : 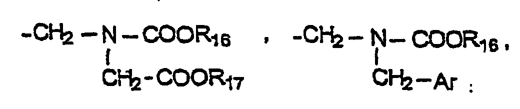 R₁₆ et R₁₇ étant choisi parmi les groupes alkyle en C₁-C₆ et Ar étant un groupe aryle en C₆-C₁₄,
et les sels d'addition de ces composés avec des acides pharmaceutiquement acceptables.

2. Composition pharmaceutique comprenant une quantité efficace d'un composé choisi parmi les composés de formule I dans laquelle :
- X est choisi parmi l'oxygène, le groupe =NH, le groupe = N-OH,
- R₁ est choisi parmi l'hydrogène, les halogènes, le groupe nitro et les groupes -NR₈R₉ dans lesquels R₈ et R₉ sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes alkyle(C₁-C₄), phénylalkyle (C₁-C₄), -(CH₂)₂-N(CH₃)₂, et -(CH₂)₂-O-(CH₂)₂-N(CH₃)₂,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
l'hydrogène, un atome d'halogène,
les groupes alkyle en C₁-C₆, hydroxyle, alcoxy en C₁-C₆, -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis indépendamment l'un de l'autre parmi l'hydrogène, les groupes alkyle (C₁-C₆) et -CH₂-CH₂-N(CH₃)₂,
les groupes -phényle-CO-CH₃ ou -phényle-CO-CH=CH-N(CH₃)₂, morpholino, nitro, SO₃H,
les groupes : 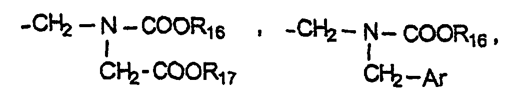 R₁₆ et R₁₇ étant choisis parmi les groupes alkyle en C₁-C₆ et Ar étant un groupe aryle en C₆-C₁₄,
et les sels d'addition de ces composés avec des acides pharmaceutiquement acceptables.

3. Composition pharmaceutique selon la revendication 2 comprenant une quantité efficace d'un composé choisi parmi les composés de formule I dans laquelle :
- X représente l'oxygène,
- R₁ est choisi parmi l'hydrogène et le groupe amino,
- R₂ est choisi parmi l'hydrogène et les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes méthyle, phénylalkyle (C₁-C₄), -(CH₂)₂-N(CH₃)₂, -(CH₂)₂-O-(CH₂)₂-N(CH₃)₂ ;
- R₄ est choisi parmi l'hydrogène, les halogènes et le groupe nitro et amino,
- R₅, R_{6 ,} R₇ représentent un hydrogène,
et leurs sels d'addition de ces composés avec des acides pharmaceutiquement acceptables.

4. Composition pharmaceutique selon la revendication 1 comprenant une quantité efficace d'un composé choisi parmi les composés de formules I et la dans lesquelles :
- X représente l'oxygène,
- R₁ est choisi parmi l'hydrogène et le groupe amino,
- R₂ est choisi parmi l'hydrogène et les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes méthyle, phénylalkyle (C₁-C₄) et les groupes -(CH₂)ₙ-Y avec Y est choisi parmi les halogènes et les groupes CN, -CH(O-Et)₂, alcoxy(C₁-C₆),-O-(CH₂)₂-N(CH₃)₂, -N(CH₃)₂ et n = 1 à 3,
- R₄ est choisi parmi l'hydrogène, les halogènes et les groupes nitro et amino,
- R₅ est choisi parmi un hydrogène, un halogène et un groupe méthoxy,
- R₆, R₇ sont choisis parmi l'hydrogène, les groupes alcoxy(C₁-C₆) et alcoxy (C₁-C₆)alkyle(C₁-C₆) et -CH₂OCOCH₃,
et les sels d'addition de ces composés avec des acides pharmaceutiquement acceptables.

5. Composition selon la revendication 4 dans laquelle les composés sont choisis parmi :
la 5-(diméthylamino)-9*H*-quino[4,3,2-*de*][1,10]phénanthrolin-9-one,
la 5-(benzylamino)-9*H*-quino[4,3,2-*de*][1,10]phénanthrolin-9-one,
la 5-bromo-9*H*-quino[4,3,2-*de*][1,10]phénantrolin-9-one,
la 5-amino-9*H*-quino[4,3,2-*de*][1,10]phénanthrolin-9-one,
la 5-méthyl-9*H*-quino[4,3,2-*de*][1,10]phénanthrolin-9-one,
la 5-méthoxy-9*H*-quino[4,3,2-*de*][1,10]phénanthrolin-9-one,
la 5-chloro-9*H*-quino[4,3,2-*de*] [1,10]phénantrolin-9-one,
la 5-bromo-10 méthoxy-9*H*-quino[4,3,2-*de*][1,10]phénanthrolin-9-one,
la 5-(diméthylamino-2-éthyl)amino-9H-quino[4,3,2de][1,10]phénanthrolin-9-one,
la 5-bis(2-chloroéthyl)amino-9*H*-quino[4,3,2de][1,10]phénanthrolin-9-one,
la 5-(2-chloroéthyl)amino-9*H*-quino[4,3,2de][1,10]phénanthrolin-9-one,
la 4-bromo-5-amino-9-*H*-quino[4,3,2-*de*][1,10] phénanthrolin-9-one,
la 5-bromo-9-*H*-quino[4,3,2-*de*][1,7]phénanthrolin-9-one,
la 5-amino-9-*H*-quino[4,3,2-*de*][1,7]phénanthrolin-9-one,
la 5-(diméthylamino-2-éthyl)amino-9-*H*-quino[4,3,2-*de*][1,7]phénanthrolin-9-one,
la 5-bis(chloroéthylamino-2-éthyl)amino-9-*H*-quino[4,3,2-*de*][1,7]phénanthrolin-9-one,
la 5-(chloroéthylamino-2-éthyl)amino-9-*H*-quino[4,3,2-*de*][1,7]phénanthrolin-9-one,
la 4-bromo-5-amino-9-*H*-quino[4,3,2-*de*][1,7]phénanthrolin-9-one,
et leurs sels d'addition avec des acides pharmaceutiquement acceptables.

6. Utilisation d'un composé tel que défini à l'une des revendications 1 à 5 pour la fabrication d'un médicament anticancéreux.

7. Utilisation selon la revendication 6 dans laquelle les composés sont choisis parmi:
la 5-(diméthylamino)-9*H*-quino[4,3,2-*de*][1,10]phénanthrolin-9-one,
la 5-(benzylamino)-9*H*-quino[4,3,2-*de*][1,10]phénanthrolin-9-one,
la 5-bromo-9*H*-quino[4,3,2-*de*][1,10]phénantrolin-9-one,
la 5-amino-9*H*-quino[4,3,2-*de*][1,10]phénanthrolin-9-one,
la 5-méthyl-9*H*-quino[4,3,2-*de*][1,10]phénanthrolin-9-one,
la 5-méthoxy-9*H*-quino[4,3,2-*de*][1,10]phénanthrolin-9-one,
la 5-chloro-9*H*-quino[4,3,2-*de*][1,10]phénantrolin-9-one,
la 5-bromo-10 méthoxy-9*H*-quino[4,3,2-*de*][1,10]phénanthrolin-9-one,
la 5-(diméthylamino-2-éthyl)amino-9*H*-quino[4,3,2de][1,10]phénanthrolin-9-one,
la 5-bis(2-chloroéthyl)amino-9*H*-quino[4,3,2de][1,10]phénanthrolin-9-one,
la 5-(2-chloroéthyl)amino-9*H*-quino[4,3,2de][1,10]phénanthrolin-9-one,
la 4-bromo-5-amino-9-*H*-quino[4,3,2-de][1,10]phénanthrolin-9-one,
la 5-bromo-9-*H*-quino[4,3,2-*de*][1,7]phénanthrolin-9-one,
la 5-amino-9-*H*-quino[4,3,2-*de*][1,7]phénanthrolin-9-one,
la 5-(diméthylamino-2-éthyl)amino-9-*H*-quino[4,3,2-*de*][1,7]phénanthrolin-9-one,
la 5-bis(chloroéthylamino-2-éthyl)amino-9-*H*-quino[4,3,2-*de*][1,7]phénanthrolin-9-one,
la 5-(chloroéthylamino-2-éthyl)amino-9-*H*-quino[4,3,2-*de*][1,7]phénanthrolin-9-one,
la 4-bromo-5-amino-9-*H*-quino[4,3,2-*de*][1,7]phénanthrolin-9-one,
et leurs sels d'addition avec des acides pharmaceutiquement acceptables.

8. Composés de formules générales I et la 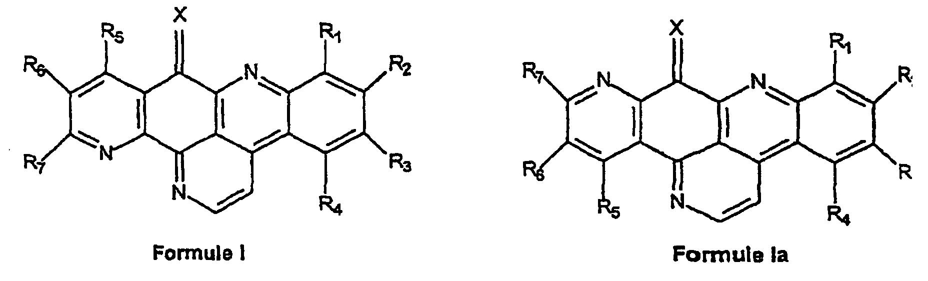 dans lesquelles :
- X est choisi parmi l'oxygène, le groupe =NH, le groupe = N-OH,
- R₁ est choisi parmi l'hydrogène, les halogènes, le groupe nitro et les groupes -NR₈R₉ dans lesquels R₈ et R₉ sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes alkyle (C₁-C₄), phénylalkyle (C₁-C₄), et les groupes -(CH₂)ₙ-Y avec Y est choisi parmi les halogènes et les groupes CN, -CH(O-Et)₂, alcoxy(C₁-C₆), -O-(CH₂)₂-N(CH₃)₂, -N(CH₃)₂ et n = 1 à 3,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
l'hydrogène, un atome d'halogène,
les groupes alkyle en C₁-C₆, hydroxyle, alcoxy en C₁-C₆, alcoxy(C₁-C₆)alkyle(C₁-C₆), alkyl(C₁-C₄) carbonyloxyalkyle(C₁-C₄), -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis indépendamment l'un de l'autre parmi l'hydrogène, les groupes alkyle (C₁-C₆), -phényle-CO-CH₃ et -CH₂-CH₂-N(CH₃)₂,
les groupes -phényle-CO-CH₃ ou -phényle-CO-CH=CH-N(CH₃)₂, morpholino, nitro, SO₃H,
les groupes : 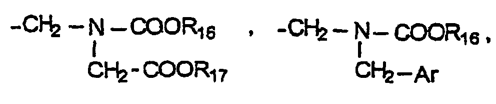 R₁₆ et R₁₇ étant choisis parmi les groupes alkyle en C₁-C₆ et Ar étant un groupe aryle en C₆-C₁₄,
à l'exclusion des composés de formule I dans laquelle X = O, et R₁, R₂, R₄, R₅, R₆, R₇ = H et R₃ = OCH₃
et leurs sels d'addition avec des acides pharmaceutiquement acceptables.

9. Composés selon la revendication 8 de formule I dans laquelle :
- X est choisi parmi l'oxygène, le groupe =NH, le groupe = N-OH,
- R₁ est choisi parmi l'hydrogène, les halogènes, le groupe nitro et les groupes -NR₈R₉ dans lesquels R₈ et R₉ sont choisis indépendamment l'un de l'autre parmi l'hydrogène et les groupes alkyle (C₁-C₄) ;
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes alkyle (C₁-C₄), phénylalkyle (C₁-C₄), -(CH₂)₂-N(CH₃)₂, et -(CH₂)₂-O-(CH₂)₂-N(CH₃)₂,
- R₄ est choisi parmi l'hydrogène, les halogènes, le groupes nitro, et les groupes -NR₁₂R₁₃ dans lesquels R₁₂ et R₁₃ sont choisis, indépendamment l'un de l'autre, parmi l'hydrogène et les groupes alkyle (C₁-C₄) ;
- R₅, R₆ et R₇ sont choisis parmi :
l'hydrogène, un atome d'halogène,
les groupes alkyle en C₁-C₆, hydroxyle, alcoxy en C₁-C₆, -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis, indépendamment l'un de l'autre, parmi l'hydrogène, les groupes alkyle (C₁-C₆) et -CH₂-CH₂-N(CH₃)₂,
les groupes. -phényl-CO-CH₃ ou -phényl-CO-CH=CH-N(CH₃)₂, morpholino, nitro, SO₃H,
les groupes : 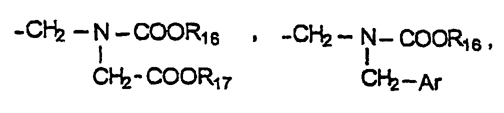 R₁₆ et R₁₇ étant choisis parmi les groupes alkyle en C₁-C₆ et Ar étant un groupe aryle en C₆-C₁₄,
à l'exclusion des composés dans lesquels X = O, et R₁, R₂, R₄, R₅, R₆, R₇ = H et R₃ = OCH₃,
et leurs sels d'addition avec des acides pharmaceutiquement acceptables.

10. Composés selon la revendication 8 qui sont :
la 5-(diméthylamino)-9*H*-quino[4,3,2-*de*][1,10]phénanthrolin-9-one,
la 5-(benzylamino)-9*H*-quino[4,3,2-*de*][1,10]phénanthrolin-9-one,
la 5-bromo-9*H*-quino[4,3,2-*de*][1,10]phénantrolin-9-one,
la 5-amino-9*H*-quino[4,3,2-*de*][1,10]phénanthrolin-9-one,
la 5-méthyl-9*H*-quino[4,3,2-*de*][1,10]phénanthrolin-9-one,
la 5-chloro-9*H*-quino [4,3,2-*de*] [1,10] phénantrolin-9-one,
la 5-bromo-10 méthoxy-9*H*-quino[4,3,2-*de*][1,10] phénanthrolin-9-one,
la 5-(diméthylamino-2-éthyl)amino-9H-quino[4,3,2de][1,10]phénarithrolin-9-one,
la 5-bis(2-chloroéthyl)amino-9*H*-quino[4,3,2de][1,10]phénanthrolin-9-one,
la 5-(2-chloroéthyl)amino-9*H*-quino[4,3,2de][1,10]phénanthrolin-9-one,
la 4-bromo-5-amino-9-*H*-quino[4,3,2-*de*][1,10] phénanthrolin-9-one,
la 5-bromo-9-*H*-quino[4,3,2-*de*][1,7]phénanthrolin-9-one,
la 5-amino-9-*H*-quino[4,3,2-*de*][1,7]phénanthrolin-9-one,
la 5-(diméthylamino-2-éthyl)amino-9-*H*-quino[4,3,2-*de*][1,7]phénanthrolin-9-one,
la 5-bis(chloroéthylamino-2-éthyl)amino-9-*H*-quino[4,3,2-*de*][1,7]phénanthrolin-9-one,
la 5-(chloroéthylamino-2-éthyl)amino-9-*H*-quino[4,3,2-*de*][1,7]phénanthrolin-9-one,
la 4-bromo-5-amino-9-*H*-quino[4,3,2-*de*][1,7]phénanthrolin-9-one,
et leurs sels d'addition avec des acides pharmaceutiquement acceptables.

11. Procédé de préparation d'un composé de formule la dans laquelle :
- X est choisi parmi l'oxygène, le groupe =NH, le groupe = N-OH,
- R₁ est choisi parmi l'hydrogène, les halogènes, le groupe, nitro et les groupes -NR₈R₉ dans lesquels R₈ et R₉ sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes alkyle (C₁-C₄), phénylalkyle (C₁-C₄), et les groupes -(CH₂)ₙ-Y avec Y est choisi parmi les halogènes et les groupes CN, -CH(O-Et)₂, alcoxy(C₁-C₆), -O-(CH₂)₂-N(CH₃)₂, -N(CH₃)₂ et n = 1 à 3,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
l'hydrogène, un atome d'halogène,
les groupes alkyle en C₁-C₆, hydroxyle, alcoxy en C₁-C₆, alcoxy(C₁-C₆)alkyle(C₁-C₆), alkyl(C₁-C₄) carbonyloxyalkyle(C₁-C₄), -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis indépendamment l'un de l'autre parmi l'hydrogène, les groupes alkyle (C₁-C₆), -phényle-CO-CH₃ et -CH₂-CH₂-N(CH₃)₂,
les groupes -phényle-CO-CH₃ ou -phényle-CO-CH=CH-N(CH₃)₂, morpholino, nitro, SO₃H,
les groupes : 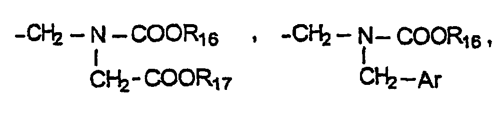 R₁₆ et R₁₇ étant choisis parmi les groupes alkyle en C₁-C₆ et Ar étant un groupe aryle en C₆-C₁₄,
qui consiste à :
a - condenser un acide chlorobenzoïque de formule : 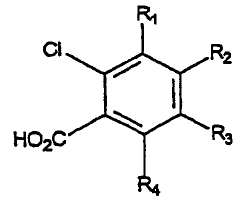 avec une diméthoxyaniline de formule: 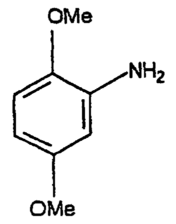 pour obtenir un composé de formule IIa : 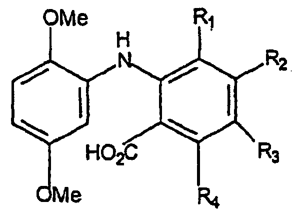
b - cycliser le composé de formule IIa pour obtenir un composé de formule 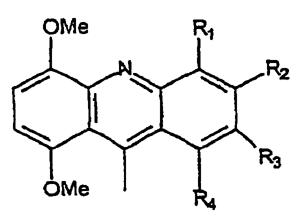
c - convertir le composé en quinone de formule IIIa : 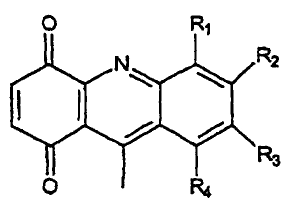
d - faire réagir la quinone de formule IIIa avec un azadiène de formule : 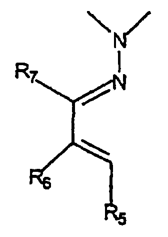 pour obtenir un composé de formule IVa : 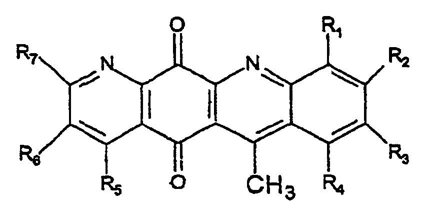
e - faire réagir le composé de formule IVa avec le diéthylacétal du diméthylformamide pour obtenir le composé de formule la,
f - et, éventuellement, convertir le composé ainsi obtenu en un autre composé de formule la.

12. Procédé de préparation de composés de formule générale I de formule : 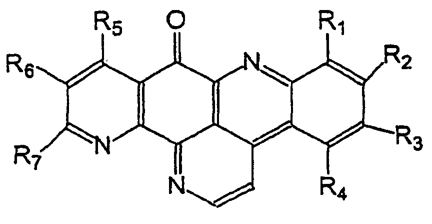 dans laquelle :
- R₁ est choisi parmi l'hydrogène, les halogènes, le groupe nitro et les groupes -NR₈R₉ dans lesquels R₈ et R₉ sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes alkyle (C₁-C₄), phénylalkyle (C₁-C₄), et les groupes -(CH₂)ₙ-Y avec Y est choisi parmi les halogènes et les groupes CN, -CH(O-Et)₂, alcoxy(C₁-C₆), -O-(CH₂)₂-N(CH₃)₂, -N(CH₃)₂ et n =1 à 3,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
l'hydrogène, un atome d'halogène,
les groupes alkyle en C₁-C₆, hydroxyle, alcoxy en C₁-C₆, alcoxy(C₁-C₆)alkyle(C₁-C₆), alkyl(C₁-C₄) carbonyloxyalkyle(C₁-C₄), -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis indépendamment l'un de l'autre parmi l'hydrogène, les groupes alkyle (C₁-C₆), -phényle-CO-CH₃ et -CH₂-CH₂-N(CH₃)₂,
les groupes -phényle-CO-CH₃ ou -phényle-CO-CH=CH-N(CH₃)₂, morpholino, nitro, SO₃H,
les groupes : 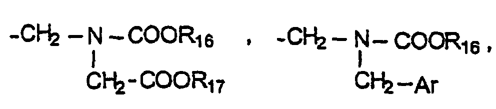 R₁₆ et R₁₇ étant choisis parmi les groupes alkyle en C₁-C₆ et Ar étant un groupe aryle en C₆-C₁₄,
à l'exclusion des composés de formule I dans laquelle R₁, R₂, R₄, R₅, R₆, R₇ = H et R₃ = OCH₃, qui consiste
a) à faire réagir une hydroquinone de formule 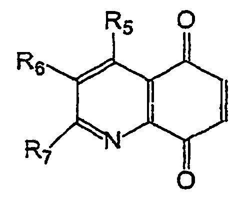 avec un composé de formule 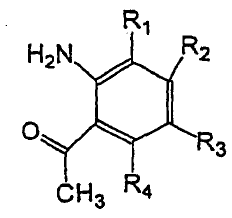 en présence de CeCl₃, 7H₂O et d'éthanol pour obtenir un composé de formule II 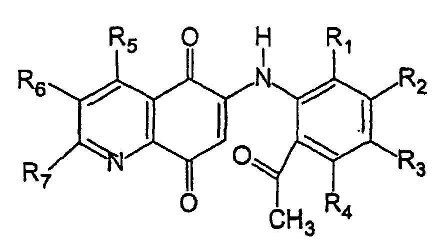
b) à transformer le composé de formule II en un composé de formule III 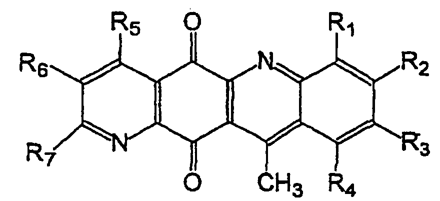 en présence de H₂SO₄ concentré dans l'acide acétique au reflux
c) à faire réagir le composé de formule III avec NC(OC₂H₅)₂ N(CH₃)₂ dans le DMF à 120°C pour former un composé de formule IV 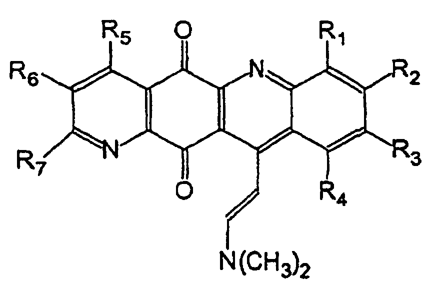
d) à cycliser le composé de formule IV en un composé de formule I en présence de NH₄Cl et AcOH.
e) éventuellement convertir le composé de formule I ainsi obtenu en un autre composé de formule I.

13. Composé de formule 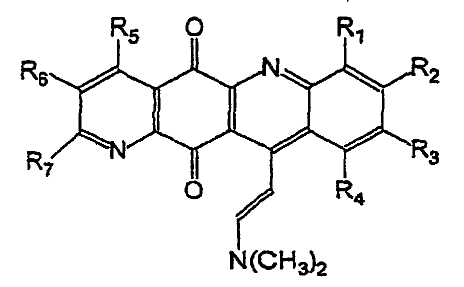 dans laquelle
- R₁ est choisi parmi l'hydrogène, les halogènes, le groupe nitro et les groupes -NR₈R₉ dans lesquels R₈ et R₉ sont choisis indépendamment l'un de l'autre parmi l'hydrogène et les groupes alkyle (C₁-C₄),
- R₂ est choisi parmi l'hydrogène, les halogènes,
- R₃ est choisi parmi les halogènes, les groupes alkyle (C₁-C₄), les groupes alcoxy (C₁-C₆), un groupe guanidino, les groupes -NR₁₀R₁₁ dans lesquels R₁₀ et R₁₁ sont choisis, indépendamment l'un de l'autre, parmi l'hydrogène, les groupes alkyle (C₁-C₄), phénylalkyle (C₁-C₄), et les groupes -(CH₂)ₙ-Y avec Y est choisi parmi les halogènes et les groupes CN, -CH(O-Et)₂, alcoxy(C₁-C₆), -O-(CH₂)₂-N(CH₃)₂, -N(CH₃)₂ et n = 1 à 3,
- R₄ est choisi parmi l'hydrogène, les halogènes, les groupes nitro, et les groupes -NR₁₂R₁₃ dans lesquels R₁₂ et R₁₃ sont choisis indépendamment l'un de l'autre parmi l'hydrogène et les groupes alkyle (C₁-C₄),
- R₅, R₆ et R₇ sont choisis parmi :
l'hydrogène, un atome d'halogène,
les groupes alkyle en C₁-C₆, hydroxyle, alcoxy en C₁-C₆, alcoxy(C₁-C₆)alkyle(C₁-C₆), alkyl(C₁-C₄) carbonyloxyalkyle(C₁-C₄), -CHO, -COOH, -CN, -CO₂R₁₄, -CONHR₁₄, -CONR₁₄R₁₅, les groupes -NHCOR₁₄ et -NR₁₄R₁₅, dans lesquels R₁₄ et R₁₅ sont choisis indépendamment l'un de l'autre parmi l'hydrogène, les groupes alkyle (C₁-C₆), ―phényle-CO-CH₃ et -CH₂-CH₂-N(CH₃)₂,
les groupes ―phényle-CO-CH₃ ou ―phényle-CO-CH=CH―N(CH₃)₂, morpholino, nitro, SO₃H,
les groupes : 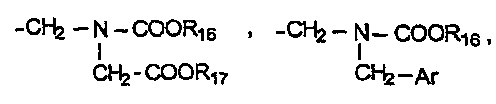 R₁₆ et R₁₇ étant choisis parmi les groupes alkyle en C₁-C₆ et Ar étant un groupe aryle en C₆-C₁₄,
à l'exclusion des composés dans lesquels ou bien R₁, R₂, R₃, R₄, R₅, R₆, R₇ = H, ou bien R₁, R₃, R₄, R₅, R₆, R₇ = H et R₂ = Br, ou bien R₁, R₂, R₄, R₅, R₆, R₇ = H et R₃ = OCH₃,ou bien R₁, R₂, R₃, R₄, R₆, R₇ = H et R₅ = OH ou OCH₃, ou bien R₁,= NO₂ et R₂, R₃, R₄, R₅, R₆, R₇ = H,
et leurs sels d'addition avec des acides pharmaceutiquement acceptables.

## Patentansprüche

1. Pharmazeutische Zubereitung, enthaltend eine wirksame Menge einer Verbindung, ausgewählt aus den zytotoxische Eigenschaften aufweisenden Verbindungen der Formeln I und Ia zur Behandlung von cancerogenen Tumoren und deren Metastasen: 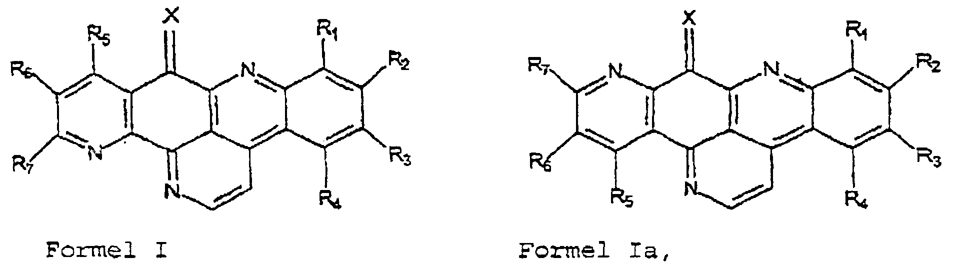 worin :
- X ausgewählt ist aus Sauerstoff, der Gruppe =NH, der Gruppe =N-OH,
- R₁ ausgewählt ist aus Wasserstoff, den Halogenen, der Nitrogruppe und den Gruppen -NR₈R₉, in denen R₈ und R₉ unabhängig voneinander ausgewählt sind aus Wasserstoff und C₁-C₄-Alkylgruppen,
- R₂ ausgewählt ist aus Wasserstoff, den Halogenen,
- R₃ ausgewählt ist aus den Halogenen, den C₁-C₄-Alkylgruppen den C₁-C₆-Alkoxygruppen einer Guanidin-Gruppe, den Gruppen NR₁₀R₁₁, in denen R₁₀ und R₁₁ unabhängig voneinander ausgewählt sind aus Wasserstoff, den C₁-C₄-Alkylgruppen, C₁-C₄-Phenylalkyl, und den Gruppen -(CH₂)ₙ-Y, wobei Y ausgewählt ist aus den Halogenen und den Gruppen CN, -CH(O-Et)₂, C₁-C₆-Alkoxy, -O-(CH₂)₂-N(CH₃)₂, -N(CH₃)₂ worin n 1 bis 3 beträgt,
- R₄ ausgewählt ist aus Wasserstoff, den Halogenen, den Nitrogruppen und den Gruppen NR₁₂R₁₃, in denen R₁₂ und R₁₃ unabhängig voneinander ausgewählt sind aus Wasserstoff und den C₁-C₄-Alkylgruppen,
- R₅, R₆ und R₇ ausgewählt sind aus Wasserstoff, einem Halogenatom, den C₁-C₆-Alkylgruppen, Hydroxyl, C₁-C₆-Alkoxy, C₁-C₆-Alkoxyalkyl (C₁-C₆), C₁-C₄-Alkylcarbonyloxyalkyl (C₁-C₄), -CHO, -COOH, -CN, - CO₂R₁₄, -CONHR₁₄, -CONR₁₄R₁₅, den Gruppen
- NHCOR₁₄ und -NR₁₄R₁₅, in denen R₁₄ und R₁₅ unabhängig voneinander ausgewählt sind aus Wasserstoff, den C₁-C₆-Alkylgruppen, -Phenyl-CO-CH₃ und -CH₂-CH₂-N(CH₃)₂,
- den Gruppen Phenyl-CO-CH₃ oder -Phenyl-CO-CH=CH-N(CH₃)₂, Morpholino, Nitro, SO₃H,
- den Gruppen 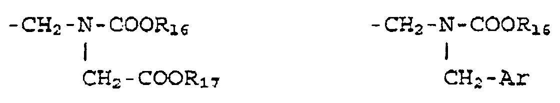 R₁₆ und R₁₇ ausgewählt sind aus den C₁-C₆ Alkylgruppen und Ar eine Arylgruppe mit 6 bis 14 Kohlenstoffatomen ist,
und die Additionssalze dieser Verbindungen mit pharmazeutisch verträglichen Säuren.

2. Pharmazeutische Zubereitungen, enthaltend eine wirksame Menge einer Verbindung, ausgewählt aus den Verbindungen der Formel I, worin:
- X ausgewählt ist aus Sauerstoff, der Gruppe =NH, der Gruppe =N-OH,
- R₁ ausgewählt ist aus Wasserstoff, den Halogenen, der Nitrogruppe und den Gruppen -NR₈R₉, in denen R₈ und R₉ unabhängig voneinander ausgewählt sind aus Wasserstoff und C₁-C₄-Alkylgruppen,
- R₂ ausgewählt ist aus Wasserstoff, den Halogenen,
- R₃ ausgewählt ist aus den Halogenen, den C₁-C₄-Alkylgruppen,den C₁-C₆-Alkoxygruppen, einer Guanidingruppe, den Gruppen NR₁₀R₁₁, in denen R₁₀ und R₁₁ unabhängig voneinander ausgewählt sind aus Wasserstoff, den C₁-C₄-Alkylgruppen, C₁-C₄-Phenylalkyl, -(CH₂)₂-N (CH₃)₂ und -(CH₂)₂-O(CH₂)₂-N(CH₃)₂,
- R₄ ausgewählt ist aus Wasserstoff, den Halogenen, den Nitrogruppen und den Gruppen NR₁₂R₁₃, in denen R₁₂ und R₁₃ unabhängig voneinander ausgewählt sind aus Wasserstoff und den C₁-C₄-Alkylgruppen.
- R₅, R₆ und R₇ ausgewählt sind aus Wasserstoff, einem Halogenatom, den C₁-C₆-Alkylgruppen, Hydroxyl, C₁-C₆-Alkoxy, -CHO, -COOH, -CN, -CO₂R₁₄, -CONR₁₄, -CONR₁₄R₁₅, den Gruppen -NHCOR₁₄ und -NR₁₄R₁₅-Gruppen, in denen R₁₄ und R₁₅ unabhängig voneinander ausgewählt sind aus Wasserstoff, den C₂-C₆-Alkylgruppen und -CH₂-CH₂-N(CH₃)₂, den Gruppen -Phenyl-CO-CH₃ oder -Phenyl-CO-CH=CH-N(CH₃)₂, Morpholino, Nitro, SO₃H, 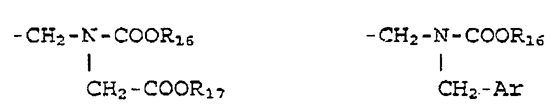 R₁₆ und R₁₇ sind ausgewählt aus den C₁-C₆-Alkylgruppen und Ar ist eine C₆-C₁₄-Arylgruppe und die Additionssalze dieser verbindungen mit pharmazeutisch verträglichen Säuren.

3. Pharmazeutische Zusammensetzung nach Anspruch 2, enthaltend eine wirksame Menge einer Verbindung, ausgewählt aus den Verbindungen der Formel I, in denen
- X Sauerstoff bedeutet
- R₁ ausgewählt ist aus Wasserstoff und der Aminogruppe,
- R₂ ausgewählt ist aus Wasserstoff und den Halogenen,
- R₃ ausgewählt ist aus den Halogenen, den C₁-C₄-Alkylgruppen, den C₁-C₆-Alkoxygruppen, einer Guanidingruppe, den Gruppen NR₁₀R₁₁, worin R₁₀ und R₁₂ unabhängig voneinander ausgewählt sind aus Wasserstoff, den Gruppen Methyl, Phenylalkyl (C₁-C₄), - (CH₂)₂-N(CH₃)₂, -(CH₂)₂-O-(CH₂)₂-N(CH₃)₂,
- R₄ ausgewählt ist aus Wasserstoff, den Halogenen, der Nitrogruppe und der Aminogruppe,
- R₅, R₆, R₇ Wasserstoff bedeuten,
und die Additionssalze dieser Verbindungen mit pharmazeutisch verträglichen Säuren.

4. Pharmazeutische Zubereitung nach Anspruch 1, enthaltend eine wirksame Menge einer Verbindung, ausgewählt aus den Verbindungen der Formeln I, Ia, worin
- X Sauerstoff bedeutet,
- R₁ ausgewählt ist aus Wasserstoff und der Aminogruppe,
- R₂ ausgewählt ist aus Wasserstoff und den Halogenen,
- R₃ ausgewählt ist aus den Halogenen, den C₁-C₄-Alkylgruppen, den C₁-C₆-Alkoxygruppen, einer Guanidingruppe, den - NR₁₀R₁₁-Gruppen, in denen R₁₀ und R₁₁ unabhängig voneinander ausgewählt sind aus wasserstoff, den Gruppen Methyl, Phenylalkyl (C₁-C₄) und den Gruppen -(CH₂)ₙ-Y, wobei Y ausgewählt ist aus den Halogenen und den Gruppen CN, -CH(O-Et)₂, C₁-C₆-Alkoxy, -O(CH₂)₂-N(CH₃)₂, -N(CH₃)₂ und n = 1 bis 3 ist.
- R₄ ausgewählt ist aus Wasserstoff, den Halogenen und der Nitro- und Aminogruppe,
- R₅ ausgewählt ist aus Wasserstoff, einem Halogen und einer Methoxygruppe,
- R₆, R₇ ausgewählt sind aus Wasserstoff, den C₁-C₆-Alkoxygruppen und C₁-C₆-Alkoxyalkyl (C₁-C₆) und - CH₂OCOCH₃
und den Additionssalzen dieser Verbindungen mit pharmazeutisch verträglichen Säuren.

5. Zubereitung nach Anspruch 4, worin die Verbindungen ausgewählt sind aus
5-(Dimethylamino)-9H-chino[4,3,2-de] [1,10]phenanthrolin-9-on,
5-(Benzylamino)-9H-chino[4,3,2-de] [1,10]phenanthrolin-9-on,
5-Brom-9H-chino[4,3,2-de] [1,10]phenanthrolin-9-on,
5-Amino-9H-chino[4,3,2-de] [1,10]phenanthrolin-9-on,
5-Methyl-9H-chino[4,3,2-de] [2,10]phenanthrolin-9-on,
5-Methoxy-9H-chino[4,3,2-de] [1,10]phenanthrolin-9-on,
5-Chloro-9H-chino[4,3,2-de] [1,10]phenanthrolin-9-on,
5-Brom-10-methoxy-9H-chino[4,3,2-de]-[1,10]phenanthrolin-9-on,
5-(Dimethylamino-2-ethyl)amino-9H-chino[4,3,2-de]-[1,10]phenanthrolin-9-on,
5-Bis(2-Chlorethyl)amino-9H-chino[4,3,2-de]-[1,10]phenanthrolin-9-on,
5-(2-Chlorethyl)amino-9H-chino[4,3,2-de]-[1,10]phenanthrolin-9-on,
4-Brom-5-amino-9-H-chino[4,3,2-de] [1,10]phenanthrolin-9-on,
5-Brom-9-H-chino[4,3,2-de] [1,7]phenanthrolin-9-on,
5-Amino-9-H-chino[4,3,2-de] [1,7]phenanthrolin-9-on,
5-(Dimethylamino-2-ethyl)amino-9-H-chino[4,3,2-de]-[1,7]phenanthrolin-9-on,
5-Bis(chlorethylamino-2-ethyl)amino-9-H-chino[4,3,2-de]- [1,7]phenanthrolin-9-on,
5-(chlorethylamino-2-ethyl)amino-9-H-chino[4,3,2-de]-[1,7]phenanthrolin-9-on,
4-Brom-5-Amino-9-H-chino[4,3,2-de] [1,7]phenanthrolin-9-on
und ihre Additionssalze mit pharmazeutisch verträglichen Säuren.

6. Verwendung einer Verbindung wie sie in den Ansprüchen 1 bis 5 definiert ist, zur Herstellung eines gegen Krebs gerichteten Medikaments.

7. Verwendung nach Anspruch 6, worin die Verbindungen ausgewählt sind aus
5-(Dimethylamino)-9H-chino[4,3,2-de] [1,10]phenanthrolin-9-on,
5-(Benzylamino)-9H-chino[4,3,2-de] [1,10]phenanthrolin-9-on,
5-Brom-9H-chino[4,3,2-de] [1,10]phenanthrolin-9-on,
5-Amino-9H-chino[4,3,2-de] [1,10]phenanthrolin-9-on,
5-Methyl-9H-chino[4,3,2-de] [1,10]phenanthxolin-9-on,
5-Methoxy-9H-chino[4,3,2-de] [1,10]phenanthrolin-9-on,
5-Chlor-9H-chino[4,3,2-de] [1,10]phenanthrolin-9-on,
5-Brom-10-methoxy-9H-chino[4,3,2-de]-[1,10]phenanthrolin-9-on,
5-(Dimethylamino-2-ethyl)amino-9H-chino[4,3,2-de]-[1,10]phenanthrolin-9-on,
5-Bis(2-Chlorethyl)amino-9H-chino[4,3,2-de]-[1,10]phenanthrolin-9-on,
5-(2-chlorethyl)amino-9H-chino[4,3,2-de]-[1,10]phenanthrolin-9-on,
4-Brom-5-amino-9-H-chino[4,3,2-de] [1,10]phenanthrolin-9-on,
5-Brom-9-H-chino[4,3,2-de] [1,7]phenanthrolin-9-on,
5-Amino-9-H-chino[4,3,2-de] [1,7]phenanthrolin-9-on,
5-(Dimethylamino-2-Ethyl)amino-9-H-chino[4,3,2-de]-[1,7]phenanthrolin-9-on,
5-Bis(chlorethylamino-2-ethyl)amino-9-H-chino[4,3,2-de]- [1,7]phenanthrolin-9-on,
5-(chlorethylamino-2-ethyl)amino-9-H-chino[4,3,2-de] [1,7]phenanthrolin-9-on,
4-Brom-5-Amino-9-H-chino[4,3,2-de] [1,7]phenanthrolin-9-on
und ihre Additionssalze mit pharmazeutisch verträglichen Säuren.

8. Verbindungen der allgemeinen Formeln I, Ia 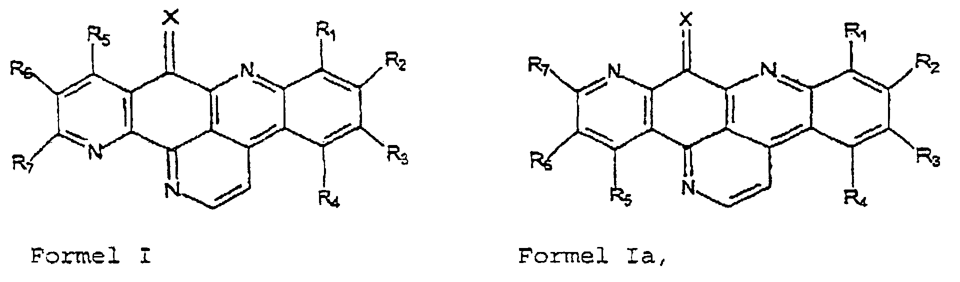 worin :
- X ausgewählt ist aus Sauerstoff, der Gruppe =NH, der Gruppe =NOH,
- R₁ ausgewählt ist aus Wasserstoff, den Halogenen, der Nitrogruppe und den Gruppen -NR₈R₉, in denen R₈ und R₉ unabhängig voneinander ausgewählt sind aus Wasserstoff und Alkylgruppen (C₁-C₄),
- R₂ ausgewählt ist aus Wasserstoff, den Halogenen,
- R₃ ausgewählt ist aus den Halogenen, den Alkylgruppen (C₁-C₄), den Alkoxygruppen (C₁-C₆), einer Guanidin-Gruppe, den Gruppen NR₁₀R₁₁, in denen R₁₀ und R₁₁ unabhängig voneinander ausgewählt sind aus Wasserstoff, den Alkylgruppen (C₁-C₄), Phenylalkyl (C₁-C₄) und den Gruppen -(CH₂)ₙ-Y, wobei Y ausgewählt ist aus den Halogenen und den Gruppen CN, -CH(O-Et)₂, Alkoxy (C₁-C₆), -O(CH₂)₂-N(CH₃)₂, -N(CH₃)₂, worin n 1-3 beträgt,
- R₄ ausgewählt ist aus Wasserstoff, den Halogenen, den Nitrogruppen und den Gruppen -NR₁₂R₁₃, in denen R₁₂ und R₁₃ unabhängig voneinander ausgewählt sind aus Wasserstoff und den Alkylgruppen (C₁-C₄),
- R₅, R₆ und R₇ ausgewählt sind aus Wasserstoff, einem Halogenatom, den C₁-C₆-Alkylgruppen, Hydroxyl, C₁-C₆-Alkoxy, C₁-C₆-Alkoxyalkyl (C₁-C₆), C₁-C₄-Alkylcarbonyloxyalkyl (C₁-C₄), -CHO, -COOH, -CN, -CO₂R₁₄, -CONHR₁₄, -CONR₁₄R₁₅, den Gruppen
- NHCOR₁₄ und -NR₁₄R₁₅, in denen R₁₄ und R₁₅ unabhängig voneinander ausgewählt sind aus Wasserstoff, den C₁-C₆-Alkylgruppen, -Phenyl-CO-CH₃ und -CH₂-CH₂-N(CH₃)₂, den Gruppen -Phenyl-CO-CH₃ oder -Phenyl-CO-CH=CH-N(CH₃)₂, Morpholino, Nitro, SO₃H, den Gruppen 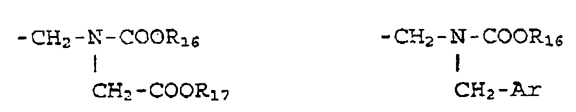 R₁₆ und R₁₇ ausgewählt sind aus den C₁-C₆ Alkylgruppen und Ar eine Arylgruppe mit 6 bis 14 Kohlenstoffatomen ist, wobei eine Verbindung der Formel I ausgeschlossen ist, in der X=O und R₁, R₂, R₄, R₅, R₆, R₇ = H und R₃ = OCH₃ ist,
und die Additionssalze dieser Verbindungen mit pharmazeutisch verträglichen Säuren.

9. Verbindungen nach Anspruch 8 der Formel I, worin
- X ausgewählt ist aus Sauerstoff, der Gruppe =NH, der Gruppe =N-OH,
- Ri ausgewählt ist aus Wasserstoff, den Halogenen, der Nitrogruppe und den Gruppen -NR₆R₉, in denen R₆ und R₉ unabhängig voneinander ausgewählt sind aus Wasserstoff und C₁-C₄-Alkylgruppen,
- R₂ ausgewählt ist aus Wasserstoff, den Halogenen,
- R₃ ausgewählt ist aus den Halogenen, den C₁-C₄-Alkylgruppen, den C₁-C₆-Alkoxygruppen, einer Guanidingruppe, den Gruppen NR₁₀R₁₁, in denen R₁₀ und R₁₁ unabhängig voneinander ausgewählt sind aus Wasserstoff, den C₁-C₄-Alkylgruppen, C₁-C₄-Phenylalkyl, -(CH₂)₂-N(CH₃)₂ und -(CH₂)₂-O(CH₂)₂-N(CH₃)₂,
- R₄ ausgewählt ist aus Wasserstoff, den Halogenen, der Nitrogruppe und den Gruppen NR₁₂R₁₃, in denen R₁₂ und R₁₃ unabhängig voneinander ausgewählt sind aus Wasserstoff und den C₁-C₄-Alkylgruppen,
- R₅, R₆ und R₇ ausgewählt sind aus Wasserstoff, einem Halogenatom, den C₁-C₆-Alkylgruppen, Hydroxyl, C₁-C₆-Alkoxy, -CHO, -COOH, -CN, -CO₂R₁₄, -CONHR₁₄, -CONR₁₄R₁₅, den Gruppen -NHCOR₁₄ und -NR₁₄R₁₅-Gruppen, in denen R₁₄ und R₁₅ unabhängig voneinander ausgewählt sind aus Wasserstoff, den C₁-C₆-Alkylgruppen und -CH₂-CH₂-N(CH₃)₂,
den Gruppen Phenyl-CO-CH₃ oder Phenyl-CO-CH=CH-N(CH₃)₂, Morpholino, Nitro, SO₃H,
den Gruppen 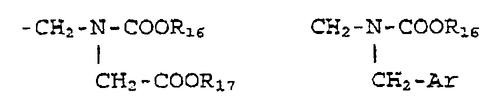 R₁₆ und R₁₇ sind ausgewählt aus den C₁-C₆-Alkylgruppen und Ar ist eine C₆-C₁₄-Arylgruppe,
wobei Verbindungen ausgeschlossen sind, in denen X = O und R₁, R₂, R₄, R₅, R₆, R₇ = H und R₃ = OCH₃ sind und ihre Additionssalze mit pharmazeutisch verträglichen Säuren.

10. Verbindungen nach Anspruch 8, wobei die Verbindungen auswählt sind aus
5-(Dimethylamino)-9H-chino[4,3,2-de] [1,10]phenanthrolin-9-on,
5-(Benzylamino)-9H-chino[4,3,2-de] [1,10]phenanthrolin-9-on,
5-Brom-9H-chino[4,3,2-de] [1,10]phenanthrolin-9-on,
5-Amino-9H-chino[4,3,2-de] [1,10]phenanthrolin-9-on,
5-Methyl-9H-chino[4,3,2-de] [1,10]phenanthrolin-9-on,
5-Chlor-9H-chino[4,3,2-de] [1,10]phenanthrolin-9-on,
5-Brom-10-methoxy-9H-chino[4,3,2-de]-[1,10]phenanthrolin-9-on,
5-(Dimethylamino-2-ethyl)amino-9H-chino[4,3,2-de]-[1,10]phenanthrolin-9-on,
5-Bis(2-Chlorethyl)amino-9H-chino[4,3,2-de][1,10]phenanthrolin-9-on,
5-(2-chlorethyl)amino-9*H*-chino[4,3,2-de][1,10]phenanthrolin-9-on
4-Brom-5-amino-9-H-chino[4,3,2-de] [1,10]phenanthrolin-9-on,
5-Brom-9-H-chino[4,3,2-de] [1,7]phenanthrolin-9-on,
5-Amino-9-H-chino[4,3,2-de] [1,7]phenanthrolin-9-on,
5-(Dimethylamino-2-Ethyl)amino-9-H-chino[4,3,2-de]-[1,7]phenanthrolin-9-on,
5-Bis(chlorethylamino-2-ethyl)amino-9-H-chino[4,3,2-de]- [1,7]phenanthrolin-9-on,
5-(chlorethylamino-2-ethyl) amino-9-H-chino[4,3,2-de]-[1,7]phenanthrolin-9-on,
4-Brom-5-Amino-9-H-chino[4,3,2-de] [1,7]phenanthrolin-9-on,
und ihre Additionssalze mit pharmazeutisch verträglichen Säuren.

11. Verfahren zur Herstellung einer Verbindung der Formel Ia, worin
- X ausgewählt ist aus Sauerstoff, der Gruppe =NH, der Gruppe =N-OH,
- R₁ ausgewählt ist aus Wasserstoff, den Halogenen, der Nitrogruppe und den -NR₈R₉, in denen R₈ und R₉ unabhängig voneinander ausgewählt sind aus Wasserstoff und den C₁-C₄-Alkylgruppen,
- R₂ ausgewählt ist aus Wasserstoff, den Halogenen,
- R₃ ausgewählt ist aus den Halogenen, den C₁-C₄-Alkylgruppen, den C₁-C₆-Alkoxygruppen, einer Guanidingruppe, den Gruppen NR₁₀R₁₁, worin R₁₀ und R₁₁ unabhängig voneinander ausgewählt sind aus Wasserstoff, den C₁-C₄-Alkylgruppen, Phenylalkyl(C₁-C₄) und den Gruppen -(CH₂)ₙ-Y mit Y ausgewählt aus den Halogenen und den Gruppen CN, -CH(O-Et)₂, C₁-C₆-Alkoxy, -O-(CH₂)-N(CH₃)₂, -N(CH₃)₂ und n = 1 bis 3 ist,
- R₄ ausgewählt ist aus Wasserstoff, den Halogenen, den Nitrogruppen und den Gruppen NR₁₂R₁₃, worin R₁₂ und R₁₃ unabhängig voneinander ausgewählt sind aus Wasserstoff und C₁-C₄-Alkylgruppen,
- R₅, R₆ und R₇ ausgewählt sind aus Wasserstoff, einem Halogenatom,den C₁-C₆-Alkylgruppen, Hydroxyl, C₁-C₆-Alkoxy, C₁-C₆-Alkoxyalkyl(C₁-C₆), C₁-C₄-Alkylcarbonyloxyalkyl(C₁-C₄), -CHO, -COOH, -CN, -CO₂R₁₄, -CONHR₁₄, -CONR₁₄R₁₅, den Gruppen -NHCOR₁₄ und -NR₁₄R₁₅, worin R₁₄ und R₁₅ unabhängig voneinander ausgewählt sind aus wasserstoff, den C₁-C₆-Alkylgruppen, -Phenyl-CO-CH₃ und -CH₂-CH₂-N(CH₃)₂,
- den Gruppen Phenyl-CO-CH₃ oder Phenyl-CO-CH=CH-N(CH₃)₂, Morpholino, Nitro, SO₃H, den Gruppen 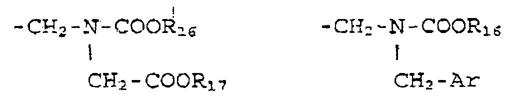
wobei R₁₆ und R₁₇ ausgewählt sind aus den C₁-C₆-Alkylgruppen, und Ar eine C₆-C₁₄-Arylgruppe ist, bestehend aus
(a) Kondensieren einer Chlorbenzolsäure der Formel 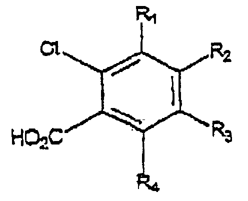 mit einem Dimethoxyanilin der Formel 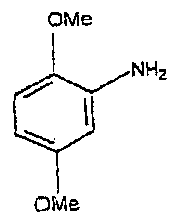 um eine Verbindung der Formel IIa 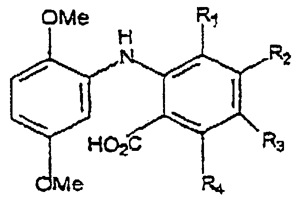 zu erhalten,
(b) Zyklisieren der Verbindung der Formel IIa, um eine Verbindung der Formel 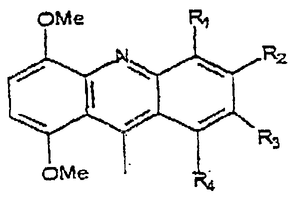 zu erhalten,
(c) Umwandeln der Verbindung in ein Chinon der Formel IIIa 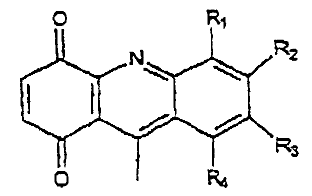
(d) Umsetzen des Chinons der Formel IIIa mit einem Azadien der Formel 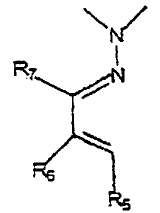 um eine Verbindung der Formel IVa zu erhalten 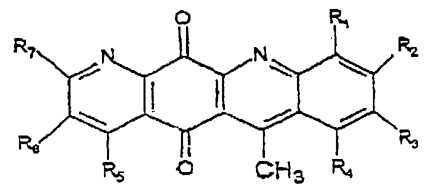
(e) umsetzen der Verbindung der Formel IVa mit einem Diethylacetal eines Dimethylformamids, um eine Verbindung der Formel Ia zu erhalten.
(f) und gegebenenfalls Umwandlung der so erhaltenen Verbindung in eine andere Verbindung der Formel Ia.

12. Verfahren zur Herstellung von Verbindungen der allgemeinen Formel I der folgenden Formel : 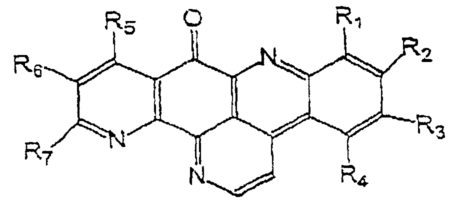 worin :
- R₁ ausgewählt ist aus Wasserstoff, den Halogenen, der Nitrogruppe und den Gruppen -NR₈R₉, in denen R₈ und R₉ unabhängig voneinander ausgewählt sind aus Wasserstoff und den C₁-C₄-Alkylgruppen,
- R₂ ausgewählt ist aus Wasserstoff, den Halogenen,
- R₃ ausgewählt ist aus den Halogenen, den C₁-C₄-Alkylgruppen, den C₁-C₆-Alkoxygruppen, einer Guanidingruppe, den Gruppen -NR₁₀R₁₁, in denen R₁₀ und R₁₁ unabhängig voneinander ausgewählt sind aus Wasserstoff, den C₁-C₄-Alkylgruppen, C₁-C₄-Phenylalkyl, und den Gruppen -(CH₂)ₙ-Y mit Y ausgewählt aus den Halogenen und den Gruppen CN, -CH(O-Et)₂, (C₁-C₆)-Alkoxy, -O-(CH₂)₂-N(CH₃)₂, N(CH₃)₂, und n = 1 bis 3,
- R₄ ausgewählt ist aus Wasserstoff, den Halogenen, den Nitrbgruppen und den Gruppen -NR₁₂R₁₃, in denen R₁₂ und R₁₃ unabhängig voneinander ausgewählt sind aus Wasserstoff und den C₁-C₄-Alkylgruppen,
- R₅, R₆ und R₇ ausgewählt sind aus:
Wasserstoff, einem Halogenatom, den Gruppen C₁-C₆-Alkyl, Hydroxyl, C₁-C₆-Alkoxy, C₁-C₆-Alkoxyalkyl(C₁-C₆), C₁-C₄-Alkylcarbonyloxyalkyl(C₁-C₄), -CHO, -COOH, -CN, -CO₂R₁₄, -CONHR₁₄, -CONR₁₄R₁₅, den Gruppen NHCOR₁₄ und -NR₁₄R₁₅, in denen R₁₄ und R₁₅ unabhängig voneinander ausgewählt sind aus Wasserstoff, den C₁-C₆-Alkylgruppen, -Phenyl-CO-CH₃ und -CH₂-CH₂-N(CH₃)₂, den Gruppen -Phenyl-CO-CH₃ oder - Phenyl-CO-CH=CH-N(CH₃)₂, Morpholino, Nitro, SO₃H, den Gruppen: 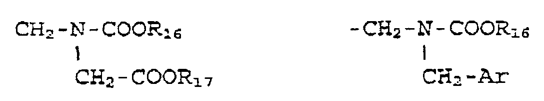 wobei R₁₆ und R₁₇ ausgewählt sind aus den C₁-C₆-Alkylgruppen und Ar eine C₆-C₁₄-Arylgruppe ist,
wobei Verbindungen der Formel I ausgeschlossen sind, in denen R₁, R₂, R₄, R₅, R₆, R₇ = H und R₃ = OCH₃, das darin besteht,
a) ein Hydrochinon der Formel 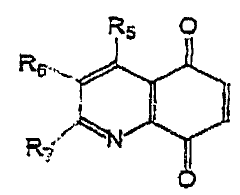 mit einer Verbindung der Formel 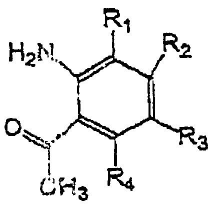 in Gegenwart von CeC₁₃, 7H₂O und Ethanol umzusetzen, um eine Verbindung der Formel II zu erhalten. 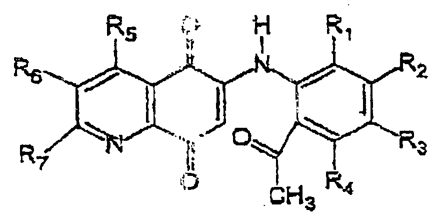
b) Die Verbindung der Formel II in Gegenwart von konzentrierter Schwefelsäure in Essigsäure unter Rückfluss in eine Verbindung der Formel III zu überführen. 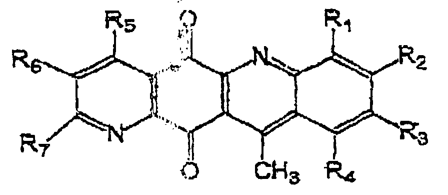
c) die Verbindung der Formel III mit HC(OC₂H₅)₂N(CH₃)₂ in DMF bei 120°C umzusetzen, um eine Verbindung der Formel IV zu bilden. 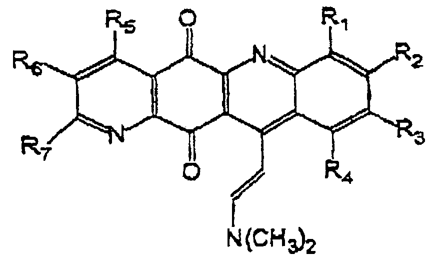
d) die Verbindung der Formel IV zu einer Verbindung der Formel I in Gegenwart von NH₄Cl und AcOH zu zyklisieren.
e) gegebenenfalls die so erhaltene Verbindung der Formel I in eine andere Verbindung der Formel I umzuwandeln.

13. Verbindung der Formel 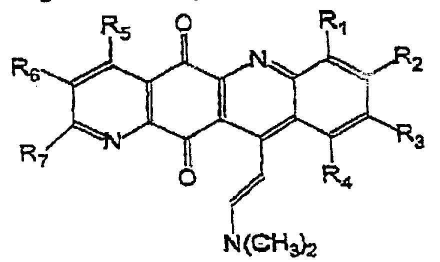 in der
- R₁ ausgewählt ist aus Wasserstoff, den Halogenen, der Nitrogruppe und den Gruppen -NR₈R₉, in denen R₈ und R₉ unabhängig voneinander ausgewählt sind aus Wasserstoff und den C₁-C₄-Alkylgruppen,
- R₂ ausgewählt ist aus Wasserstoff, den Halogenen,
- R₃ ausgewählt ist aus den Halogenen, den C₁-C₄-Alkylgruppen, den C₁-C₆-Alkoxygruppen, einer Guanidingruppe, den Gruppen -NR₁₀R₁₁, in denen R₁₀ und R₁₁ unabhängig voneinander ausgewählt sind aus Wasserstoff, den C₁-C₄-Alkylgruppen, C₁-C₄-Phenylalkyl und den Gruppen -(CH₂)ₙ-Y wobei Y ausgewählt ist aus den Halogenen und den Gruppen, CN, -CH(OEt)₂, (C₁-C₆)-Alkoxy, -O-(CH₂)₂-N(CH₃)₂, -N(CH₃)₂ und n 1 bis 3 beträgt,
- R₄ ausgewählt ist aus Wasserstoff, den Halogenen, den Nitrogruppen und den Gruppen -NR₁₂R₁₃, wobei R₁₂ und R₁₃ unabhangig voneinander ausgewählt sind aus Wasserstoff und den (C₁-C₄)-Alkylgruppen,
- R₅, R₆ und R₇ ausgewählt sind aus Wasserstoff, einem Halogenatom,
den Gruppen C₁-C₆-Alkyl, Hydroxyl, C₁-C₆-Alkoxy, C₁-C₆-Alkoxyalkyl(C₁-C₆), C₁-C₄- Alkylcarbonyloxyalkyl(C₁-C₄), -CHO, -COOH, -CN, -CO₂R₁₄, -CONHR₁₄, -CONR₁₄R₁₅, den Gruppen -NHCOR₁₄ und -NR₁₄R₁₅, in denen R₁₄ und R₁₅ unabhängig voneinander ausgewählt sind aus Wasserstoff, den C₁-C₆-Alkylgruppen, -Phenyl-CO-CH₃ und -CH₂-CH₂-N(CH₃)₂,
den Gruppen -Phenyl-CO-CH₃ oder Phenyl-CO-CH=CH-N(CH₃)₂, Morpholino, Nitro, SO₃H, den Gruppen: 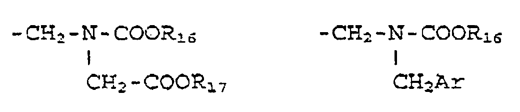 wobei R₁₆ und R₁₇ ausgewählt sind aus den C₁-C₆-Alkylgruppen und Ar eine C₆-C₁₄-Arylgruppe ist, wobei Verbindungen in denen R₁, R₂, R₃, R₄, R₅, R₆, R₇ = H, oder R₁, R₃, R₄, R₅, R₆, R₇ = H und R₂ = Br, oder R₁, R₂, R₄, R₅, R₆, R₇ = H und R₃ = OCH₃, oder R₁, R₂, R₃, R₄, R₆, R₇ = H und R₅ = OH oder OCH₃, oder R₁ = NO₂ und R₂, R₃, R₄, R₅, R₆, R₇ = H und ihre Additionssalze mit pharmazeutisch verträglichen Säuren.

## Claims

1. Pharmaceutical composition comprising an effective quantity of a compound selected from among the compounds of formula I and Ia for treating cancerous tumours and metastases thereof, by virtue of their cytotoxic properties : 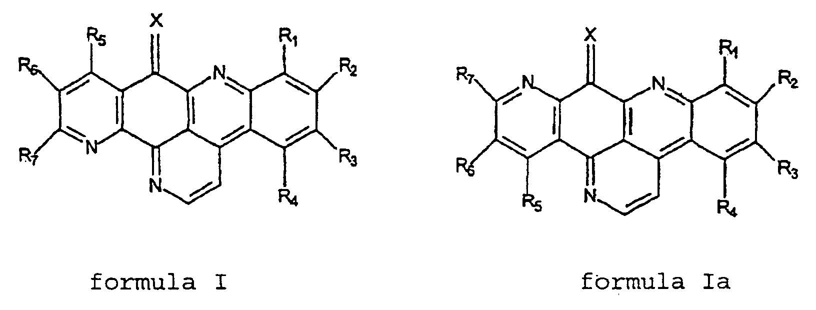 wherein :
X is selected from among oxygen, the group =NH, the group =N-OH,
R₁ is selected from among hydrogen, the halogens, the nitro group and the -NR₈R₉ groups wherein R₈ and R₉ independently of one another are selected from among hydrogen and the (C₁-C₄)alkyl groups,
R₂ is selected from among hydrogen and the halogens,
R₃ is selected from among the halogens, the (C₁-C₄)alkyl groups, the (C₁-C₆)alkoxy groups, a guanidino group, the -NR₁₀R₁₁ groups wherein R₁₀ and R₁₁ are selected independently of one another from among hydrogen, the (C₁-C₄)alkyl, phenyl (C₁-C₄)alkyl and -(CH₂)ₙ-Y groups wherein Y is selected from among the halogens and the groups, CN, -CH(O-Et)₂, (C₁-C₆)alkoxy, -O-(CH₂)₂-N(CH₃)₂, -N(CH₃)₂ and n = 1 to 3,
R₄ is selected from among hydrogen, the halogens, the nitro groups and the -NR₁₂R₁₃ groups wherein R₁₂ and R₁₃ are selected independently of one another from among hydrogen and the (C₁-C₄)alkyl groups,
R₅, R₆ and R₇ are selected from among :
hydrogen, a halogen atom,
the (C₁-C₆)alkyl, hydroxyl, (C₁-C₆)alkoxy, (C₁-C₆)alkoxy-(C₁-C₆)alkyl, (C₁-C₄)alkyl-(C₁-C₄) carbonyloxyalkyl, -CHO, -COOH, -CN, -CO₂R₁₄, -CONHR₁₄, -CONR₁₄R₁₅ groups, the groups -NHCOR₁₄ and -NR₁₄R₁₅ wherein R₁₄ and R₁₅ are selected, independently of one another, from among hydrogen, the (C₁-C₆)alkyl, -phenyl-COCH₃ and - CH₂-CH₂-N(CH₃)₂ groups,
the -phenyl-CO-CH₃ or -phenyl-CO-CH=CH-N(CH₃)₂, the morpholino, nitro, SO₃H groups,
the groups : 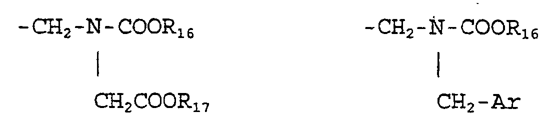
R₁₆ and R₁₇ being selected from among the (C₁-C₆)alkyl groups and Ar being a C₆-C₁₄ aryl group,
and the addition salts of these compounds with pharmaceutically acceptable acids.

2. Pharmaceutical composition comprising an effective quantity of a compound selected from among the compounds of formula I wherein:
X is selected from among oxygen, the group =NH, the group =N-OH,
R₁ is selected from among hydrogen, the halogens, the nitro group and the -NR₈R₉ groups wherein R₈ and R₉ independently of one another are selected from among hydrogen and the (C₁-C₄)alkyl groups,
R₂ is selected from among hydrogen and the halogens,
R₃ is selected from among the halogens, the (C₁-C₄)alkyl groups, the (C₁-C₆)alkoxy groups, a guanidino group, the groups -NR₁₀R₁₁ wherein R₁₀ and R₁₁ independently of one another are selected from among hydrogen, the (C₁-C₄)- alkyl, (C₁-C₄)phenylalkyl, -(CH₂)₂-N(CH₃)₂ and -(CH₂)₂-O-(CH₂)₂-N(CH₃)₂ groups,
R₄ is selected from among hydrogen, the halogens, the nitro groups and the -NR₁₂R₁₃ groups wherein R₁₂ and R₁₃ independently of one another are selected from among hydrogen and the (C₁-C₄)alkyl groups,
R₅, R₆ and R₇ are selected from among :
hydrogen, a halogen atom,
the (C₁-C₆) alkyl, hydroxyl, (C₁-C₆)alkoxy, -CHO, -COOH, -CN, -CO₂R₁₄, -CONHR₁₄, -CONR₁₄R₁₅ groups, the groups -NHCOR₁₄ and -NR₁₄R₁₅, wherein R₁₄ and R₁₅ are selected independently of one another from among hydrogen, the (C₁-C₆)alkyl and -CH₂-CH₂-N(CH₃)₂ groups,
the groups -phenyl-CO-CH₃ or - phenyl-CO-CH=CH-N(CH₃)₂ groups, the morpholino, nitro or SO₃H groups,
the groups : 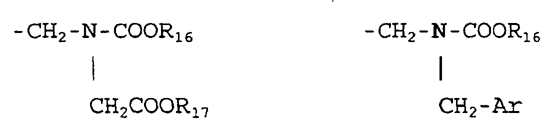
R₁₆ and R₁₇ being selected from among the (C₁-C₆)alkyl groups and Ar being a C₆-C₁₄-aryl group,
and the addition salts of these compounds with pharmaceutically acceptable acids.

3. Pharmaceutical composition according to claim 2 comprising an effective quantity of a compound selected from among the compounds of formula I wherein:
X represents oxygen,
R₁ is selected from among hydrogen and the amino group,
R₂ is selected from among hydrogen and the halogens,
R₃ is selected from among the halogens, the (C₁-C₄)alkyl groups, the (C₁-C₆)alkoxy groups, a guanidino group, the groups -NR₁₀R₁₁ wherein R₁₀ and R₁₁ are selected, independently of one another, from among hydrogen, the methyl, phenyl (C₁-C₄)alkyl, -(CH₂)₂-N(CH₃)₂ or -(CH₂)₂-O- (CH₂)₂-N(CH₃)₂ groups;
R₄ is selected from among hydrogen, the halogens and the nitro and amino group,
R₅, R₆, R₇ denote a hydrogen,
and the addition salts of these compounds with pharmaceutically acceptable acids.

4. Pharmaceutical composition according to claim 1, comprising an effective quantity of a compound selected from among the compounds of formulae I and Ia wherein :
X represents oxygen,
R₁ is selected from among hydrogen and the amino group,
R₂ is selected from among hydrogen and the halogens,
R₃ is selected from among the halogens, the (C₁-C₄)alkyl groups, the (C₁-C₆)alkoxy groups, a guanidino group, the groups -NR₁₀R₁₁ wherein R₁₀ and R₁₁ are selected, independently of one another, from among hydrogen, the methyl, phenyl (C₁-C₄)alkyl groups and the groups -(CH₂)ₙ-Y wherein Y is selected from among the halogens and the groups CN, -CH(O-Et)₂, (C₁-C₆)alkoxy, -O-(CH₂)₂-N(CH₃)₂, -N(CH₃)₂ and n = 1 to 3,
R₄ is selected from among hydrogen, the halogens and the nitro and amino groups,
R₅ is selected from among a hydrogen, a halogen and a methoxy group,
R₆ and R₇ are selected from among hydrogen, the (C₁-C₆)alkoxy groups and (C₁-C₆)alkoxy-(C₁-C₆)alkyl and - CH₂OCOCH₃ groups,
and the addition salts of these compounds with pharmaceutically acceptable acids.

5. Composition according to claim 4 wherein the compounds are selected from among :
5-(dimethylamino)-9H-quino[4,3,2-de] [1,10]phenanthrolin-9-one,
5-(benzylamino)-9H-quino[4,3,2-de] [1,10]phenanthrolin-9-one,
5-bromo-9H-quino[4,3,2-de] [1,10]phenanthrolin-9-one,
5-amino-9H-quino[4,3,2-de] [1,10]phenanthrolin-9-one,
5-methyl-9H-quino[4,3,2-de] [1,10]phenanthrolin-9-one,
5-methoxy-9H-quino[4,3,2-de] [1,10]phenanthrolin-9-one,
5-chloro-9H-quino[4,3,2-de] [1,10]phenanthrolin-9-one,
5-bromo-10 methoxy-9H-quino[4,3,2-de] [1,10]-phenanthrolin-9-one,
5-(dimethylamino-2-ethyl)amino-9H-quino[4,3,2-de] [1,10] phenanthrolin-9-one,
5-bis(2-chloroethyl)amino-9H-quino[4,3,2-de] [1,10] phenanthrolin-9-one,
5-(2-chloroethyl)amino-9H-quino[4,3,2-de] [1,10] phenanthrolin-9-one,
4-bromo-5-amino-9H-quino[4,3,2-de] [1,10]phenanthrolin-9-one,
5-bromo-9H-quino[4,3,2-de] [1,7]phenanthrolin-9-one,
5-amino-9H-quino[4,3,2-de] [1,7]phenanthrolin-9-one,
5-(dimethylamino-2-ethyl)amino-9H-quino[4,3,2-de] [1,7] phenanthrolin-9-one,
5-bis(chloroethylamino-2-ethyl)amino-9H-quino[4,3,2-de] [1,7] phenanthrolin-9-one,
5-(chloroethylamino-2-ethyl)amino-9H-quino[4,3,2-de] [1,7] phenanthrolin-9-one,
4-bromo-5-amino-9H-quino[4,3,2-de] [1,7] phenanthrolin-9-one,
and the addition salts thereof with pharmaceutically acceptable acids.

6. Use of a compound as defined in one of claims 1 to 5 for the production of an anti-cancer drug.

7. Use according to claim 6, wherein the compounds are selected from among :
5-(dimethylamino)-9H-quino[4,3,2-de] [1,10]phenanthrolin-9-one,
5- (benzylamino) -9H-quino[4,3,2-de] [1,10]phenanthrolin-9-one,
5-bromo-9H-quino[4,3,2-de] [1,10]phenanthrolin-9-one,
5-amino-9H-quino[4,3,2-de] [1,10]phenanthrolin-9-one,
5-methyl-9H-quino[4,3,2-de] [1,10]phenanthrolin-9-one,
5-methoxy-9H-quino[4,3,2-de] [1,10]phenanthrolin-9-one,
5-chloro-9H-quino[4,3,2-de] [1,10]phenanthrolin-9-one,
5-bromo-10 methoxy-9H-quino[4,3,2-de] [1,10]phenanhrolin-9-one,
5-(dimethylamino-2-ethyl)amino-9H-quino[4,3,2-de] [1,10] phenanthrolin-9-one,
5-bis(2-chloroethyl)amino-9H-quino[4,3,2-de] [1,10] phenanthrolin-9-one,
5-(2-chloroethyl)amino-9H-quino[4,3,2-de] [1,10] phenanthrolin-9-one,
4-bromo-5-amino-9H-quino[4,3,2-de] [1,10] phenanthrolin-9-one,
5-bromo-9H-quino[4,3,2-de] [1,7]phenanthrolin-9-one,
5-amino-9H-quino[4,3,2-de] [1,7]phenanthrolin-9-one,
5-(dimethylamino-2-ethyl)amino-9H-quino[4,3,2-de] [1,7] phenanthrolin-9-one,
5-bis(chloroethylamino-2-ethyl)amino-9H-quino[4,3,2-de] [1,7] phenanthrolin-9-one,
5-(chloroethylamino-2-ethyl)amino-9H-quino[4,3,2-de] [1,7] phenanthrolin-9-one,
4-bromo-5-amino-9H-quino[4,3,2-de] [1,7] phenanthrolin-9-one,
and the addition salts thereof with pharmaceutically acceptable acids.

8. Compounds of general formulae I and Ia 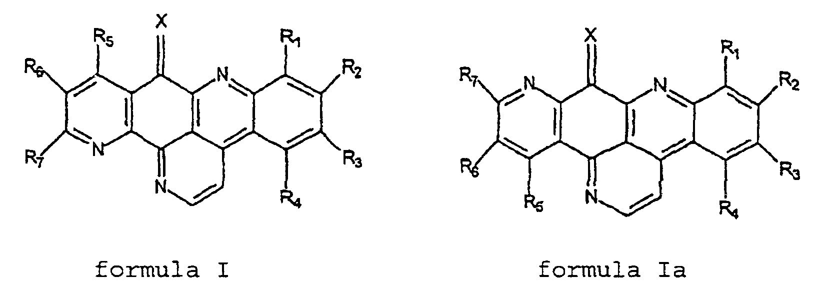 wherein :
X is selected from among oxygen, the group =NH, the group =N-OH,
R₁ is selected from among hydrogen, the halogens, the nitro group and the -NR₈R₉ groups wherein R₈ and R₉ independently of one another are selected from among hydrogen and the (C₁-C₄)alkyl groups,
R₂ is selected from among hydrogen and the halogens,
R₃ is selected from among the halogens, the (C₁-C₄)alkyl groups, the (C₁-C₆)alkoxy groups, a guanidino group, the -NR₁₀R₁₁ groups wherein R₁₀ and R₁₁ are selected independently of one another from among hydrogen, the (C₁-C₄)alkyl, phenyl (C₁-C₄)alkyl and -(CH₂)ₙ-Y groups wherein Y is selected from among the halogens and the groups -CN, -CH(O-Et)₂, (C₁-C₆)alkoxy, -O-(CH₂)₂-N(CH₃)₂, -N(CH₃)₂ and n = 1 to 3,
R₄ is selected from among hydrogen, the halogens, the nitro groups and the -NR₁₂R₁₃ groups wherein R₁₂ and R₁₃ are selected independently of one another from among hydrogen and the (C₁-C₄)alkyl groups,
R₅, R₆ and R₇ are selected from among:
hydrogen, a halogen atom,
the (C₁-C₆)alkyl, hydroxyl, (C₁-C₆)alkoxy, (C₁-C₆)alkoxy- (C₁-C₆)alkyl, (C₁-C₄)alkyl-carbonyloxy(C₁-C₄) alkyl, -CHO, -COOH, -CN, -CO₂R₁₄, -CONHR₁₄, - CONR₁₄R₁₅ groups, the groups -NHCOR₁₄ and -NR₁₄R₁₅ wherein R₁₄ and R₁₅ are selected, independently of one another, from among hydrogen, the (C₁-C₆)alkyl, -phenyl-COCH₃ and - CH₂-CH₂-N(CH₃)₂ groups,
the -phenyl-CO-CH₃ or -phenyl-CO-CH=CH-N(CH₃)₂. morpholino, nitro, SO₃H groups,
the groups : 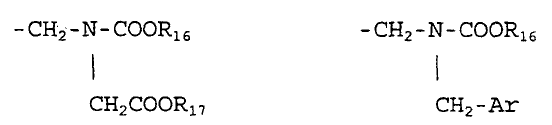
R₁₆ and R₁₇ being selected from among the (C₁-C₆)alkyl groups and Ar being a C₆-C₁₄ aryl group,
with the exception of the compound of formula Ia wherein X = O and R₁, R₂, R₄, R₅, R₆, R₇ = H and R₃ = OCH₃,
and the addition salts of these compounds with pharmaceutically acceptable acids.

9. Compounds according to claim 8 of formula I wherein :
X is selected from among oxygen, the group =NH, the group =N-OH,
R₁ is selected from among hydrogen, the halogens, the nitro group and the -NR₈R₉ groups wherein R₈ and R₉ independently of one another are selected from among hydrogen and the (C₁-C₄)alkyl groups,
R₂ is selected from among hydrogen and the halogens,
R₃ is selected from among the halogens, the (C₁-C₄)alkyl groups, the (C₁-C₆)alkoxy groups, a guanidino group, the groups -NR₁₀R₁₁ wherein R₁₀ and R₁₁ are selected independently of one another from among hydrogen, the (C₁-C₄)alkyl, (C₁-C₄)phenylalkyl, -(CH₂)₂N(CH₃)₂ and - (CH₂)₂-O-(CH₂)₂-N(CH₃)₂ groups,
R₄ is selected from among hydrogen, the halogens, the nitro groups and the -NR₁₂R₁₃ groups wherein R₁₂ and R₁₃ are selected independently of one another from among hydrogen and the (C₁-C₄)alkyl groups,
R₅, R₆ and R₇ are selected from among:
hydrogen, a halogen atom,
the (C₁-C₆)alkyl, hydroxyl, (C₁-C₆)alkoxy, -CHO, - COOH, -CN, -CO₂R₁₄, -CONHR₁₄, -CONR₁₄R₁₅ groups, the groups -NHCOR₁₄ and -NR₁₄R₁₅, wherein R₁₄ and R₁₅ are selected independently of one-another from among hydrogen, the (C₁-C₆)alkyl and -CH₂-CH₂-N(CH₃)₂ groups,
the groups -phenyl-CO-CH₃ or - phenyl-CO-CH=CH-N(CH₃)₂, morpholino, nitro or SO₃H groups,
the groups : 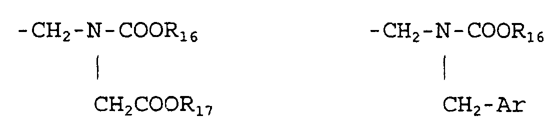
R₁₆ and R₁₇ being selected from among the C₁-C₆-alkyl groups and Ar being a C₆-C₁₄-aryl group,
with the exception of compounds wherein X = O, and R₁, R₂, R₄, R₅, R₆, R₇ = H and R₃ = OCH₃,
and the addition salts thereof with pharmaceutically acceptable acids.

10. Compounds according to claim 8 which are:
5-(dimethylamino)-9H-quino[4,3,2-de] [1,10]phenanthrolin-9-one,
5-(benzylamino)-9H-quino[4,3,2-de] [1,10]phenanthrolin-9-one,
5-bromo-9H-quino[4,3,2-de] [1,10]phenanthrolin-9-one,
5-amino-9H-quino[4,3,2-de] [1,10]phenanthrolin-9-one,
5-methyl-9H-quino[4,3,2-de] [1,10]phenanthrolin-9-one,
5-chloro-9H-quino[4,3,2-de] [1,10]phenanthrolin-9-one,
5-bromo-10-methoxy-9H-quino[4,3,2-de] [1,10]phenanhrolin-9-one,
5-(dimethylamino-2-ethyl)amino-9H-quino[4,3,2-de] [1,10] phenanthrolin-9-one,
5-bis(2-chloroethyl)amino-9H-quino[4,3,2-de] [1,10] phenanthrolin-9-one,
5-(2-chloroethyl)amino-9H-quino[4,3,2-de] [1,10] phenanthrolin-9-one,
4-bromo-5-amino-9H-quino[4,3,2-de] [1,10]phenanthrolin-9-one,
5-bromo-9H-quino[4,3,2-de] [1,7]phenanthrolin-9-one,
5-amino-9H-quino[4,3,2-de] [1,7]phenanthrolin-9-one,
5-(dimethylamino-2-ethyl)amino-9H-quino[4,3,2-de] [1,7] phenanthrolin-9-one,
5-bis(chloroethylamino-2-ethyl)amino-9H-quino[4,3,2-de] [1,7] phenanthrolin-9-one,
5-(chloroethylamino-2-ethyl)amino-9H-quino[4,3,2-de] [1,7] phenanthrolin-9-one,
4-bromo-5-amino-9H-quino[4,3,2-de] [1,7] phenanthrolin-9-one,
and the addition salts thereof with pharmaceutically acceptable acids.

11. Process for preparing a compound of formula Ia wherein :
X is selected from among oxygen, the group =NH, the group =N-OH,
R₁ is selected from among hydrogen, the halogens, the nitro group and the -NR₈R₉ groups wherein R₈ and R₉ independently of one another are selected from among hydrogen and the (C₁-C₄)alkyl groups,
R₂ is selected from among hydrogen and the halogens,
R₃ is selected from among the halogens, the (C₁-C₄)alkyl groups, the (C₁-C₆)alkoxy groups, a guanidino group, the -NR₁₀R₁₁ groups wherein R₁₀ and R₁₁ are selected independently of one another from among hydrogen, the (C₁-C₄) alkyl, phenyl (C₁-C₄)alkyl and - (CH₂)ₙ-Y groups wherein Y is selected from among the halogens and the groups, CN, -CH(O-Et)₂, (C₁-C₆)alkoxy, -O-(CH₂)₂-N(CH₃)₂, -N(CH₃)₂ and n = 1 to 3,
R₄ is selected from among hydrogen, the halogens, the nitro groups and the -NR₁₂R₁₃ groups wherein R₁₂ and R₁₃ are selected independently of one another from among hydrogen and the (C₁-C₄)alkyl groups,
R₅, R₆ and R₇ are selected from among :
hydrogen, a halogen atom,
the (C₁-C₆)alkyl, hydroxyl, (C₁-C₆)alkoxy, (C₁-C₆)alkoxy- (C₁-C₆)alkyl, (C₁-C₄)alkylcarbonyloxy(C₁-C₄)alkyl, -CHO, -COOH, -CN, -CO₂R₁₄, -CONHR₁₄, -CONR₁₄R₁₅ groups, the groups -NHCOR₁₄ and -NR₁₄R₁₅ wherein R₁₄ and R₁₅ are selected, independently of one another, from among hydrogen, the (C₁-C₆)alkyl, - phenyl-COCH₃ and -CH₂-CH₂-N(CH₃)₂ groups,
the -phenyl-CO-CH₃ or -phenyl-CO-CH=CH-N(CH₃)₂, the morpholino, nitro, SO₃H groups,
the groups : 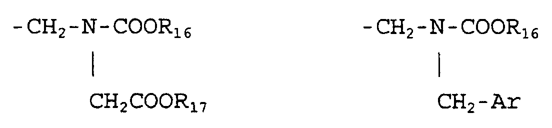
R₁₆ and R₁₇ being selected from among the (C₁-C₆)alkyl groups and Ar being a C₆-C₁₄ aryl group,
which comprises :
a- condensing a chlorobenzoic acid of formula : 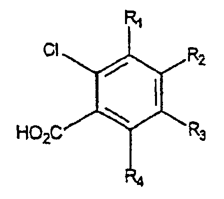 with a dimethoxyaniline of formula : 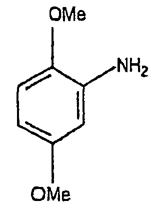 to obtain a compound of formula IIa : 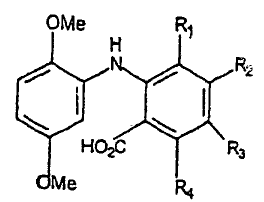
b- cyclising the compound of formula IIa to obtain a compound of formula 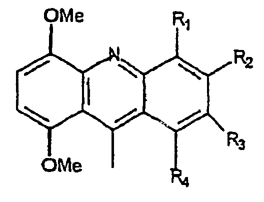
c- converting the compound into a quinone of formula IIIa: 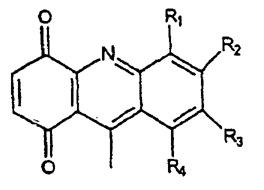
d- reacting the quinone of formula IIIa with an azadiene of formula : 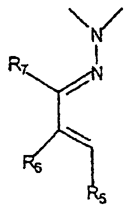 to obtain a compound of formula IVa : 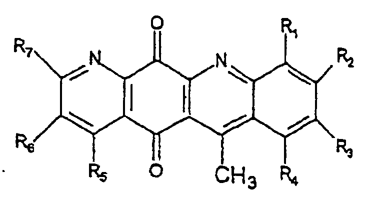
e- reacting the compound of formula IVa with the diethylacetal of dimethylformamide to obtain the compound of formula Ia,
f- and optionally converting the compound thus obtained into another compound of formula Ia.

12. Process for preparing compounds of general formula I of formula 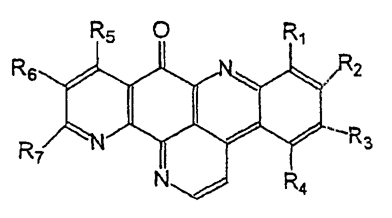 wherein :
R₁ is selected from among hydrogen, the halogens, the nitro group and the -NR₈R₉ groups wherein R₈ and R₉ independently of one another are selected from among hydrogen and the (C₁-C₄)alkyl groups,
R₂ is selected from among hydrogen and the halogens,
R₃ is selected from among the halogens, the (C₁-C₄)alkyl groups, the (C₁-C₆)alkoxy groups, a guanidino group, the -NR₁₀R₁₁ groups wherein R₁₀ and R₁₁ are selected independently of one another from among hydrogen, the (C₁-C₄)alkyl, phenyl (C₁-C₄)alkyl and -(CH₂)ₙ-Y groups wherein Y is selected from among the halogens and the groups CN, -CH(O-Et)₂, (C₁-C₆)alkoxy, -O-(CH₂)₂-N(CH₃)₂, -N(CH₃)₂ and n = 1 to 3,
R₄ is selected from among hydrogen, the halogens, the nitro groups and the -NR₁₂R₁₃ groups wherein R₁₂ and R₁₃ are selected independently of one another from among hydrogen and the (C₁-C₄)alkyl groups,
R₅, R₆ and R₇ are selected from among :
hydrogen, a halogen atom,
the (C₁-C₆)alkyl, hydroxyl, (C₁-C₆)alkoxy, (C₁-C₆)alkoxy- (C₁-C₆)alkyl, (C₁-C₄)alkyl-carbonyloxy(C₁-C₄)alkyl, -CHO, -COOH, -CN, -CO₂R₁₄, CONHR₁₄, CONR₁₄R₁₅ groups, the groups -NHCOR₁₄ and -NR₁₄R₁₅ wherein R₁₄ and R₁₅ are selected, independently of one another, from among hydrogen, the (C₁-C₆)alkyl, -phenyl-COCH₃ and - CH₂-CH₂-N(CH₃)₂ groups,
the -phenyl-CO-CH₃ or -phenyl-CO-CH=CH-N(CH₃)₂, the morpholino, nitro, SO₃H groups,
the groups : 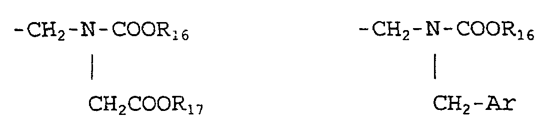
R₁₆ and R₁₇ being selected from among the (C₁-C₆)alkyl groups and Ar being a C₆-C₁₄-aryl group,
with the exception of the compounds of formula I wherein R₁, R₂, R₄, R₅, R₆, R₇ = H and R₃ = OCH₃,
which comprises
a) reacting a hydroquinone of formula 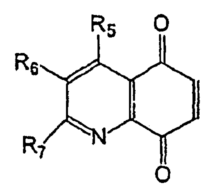 with a compound of formula 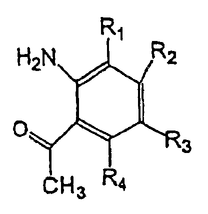 in the presence of CeCl₃. 7H₂O and ethanol to obtain a compound of formula II 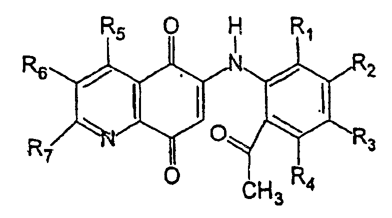
b) converting the compound of formula II into a compound of formula III 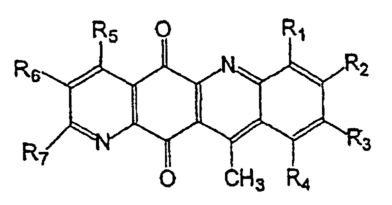 in the presence of concentrated H₂SO₄ in acetic acid at reflux temperature,
c) reacting the compound of formula III with HC(OCH₂H₅)₂ N(CH₃)₂ in DMF at 120°C to form a compound of formula IV 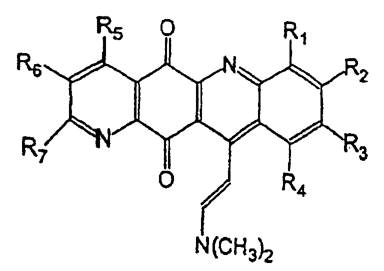
d) cyclising the compound of formula IV into a compound of formula I in the presence of NH₄Cl and AcOH,
e) optionally converting the compound of formula I thus obtained into another compound of formula I.

13. Compound of formula 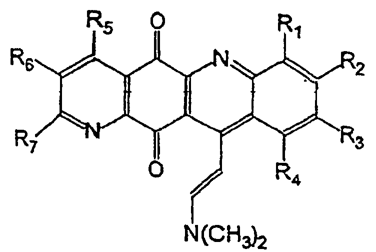 wherein
R₁ is selected from among hydrogen, the halogens, the nitro group and the -NR₈R₉ groups wherein R₈ and R₉ independently of one another are selected from among hydrogen and the (C₁-C₄)alkyl groups,
R₂ is selected from among hydrogen and the halogens,
R₃ is selected from among the halogens, the (C₁-C₄)alkyl groups, the (C₁-C₆)alkoxy groups, a guanidino group, the -NR₁₀R₁₁ groups wherein R₁₀ and R₁₁ are selected independently of one another from among hydrogen, the (C₁-C₄)alkyl, phenyl (C₁-C₄)alkyl and -(CH₂)ₙ-Y groups wherein Y is selected from among the halogens and the groups CN, -CH(O-Et)₂, (C₁-C₆)alkoxy, -O-(CH₂)₂-N(CH₃)₂, -N(CH₃)₂ and n = 1 to 3,
R₄ is selected from among hydrogen, the halogens, the nitro groups and the -NR₁₂R₁₃ groups wherein R₁₂ and R₁₃ are selected independently of one another from among hydrogen and the (C₁-C₄)alkyl groups,
R₅, R₆ and R₇ are selected from among :
hydrogen, a halogen atom,
the (C₁-C₆)alkyl, hydroxyl, (C₁-C₆)alkoxy, (C₁-C₆)alkoxy- (C₁-C₆)alkyl, (C₁-C₄)alkyl-carbonyloxy(C₁-C₄)alkyl, -CHO, -COOH, -CN, -CO₂R₁₄, -CONHR₁₄, -CONR₁₄R₁₅ groups, the groups -NHCOR₁₄ and -NR₁₄R₁₅ wherein R₁₄ and R₁₅ are selected, independently of one another, from among hydrogen, the (C₁-C₆)alkyl, -phenyl-COCH₃ and - CH₂-CH₂-N(CH₃)₂ groups,
the -phenyl-CO-CH₃ or -phenyl-CO-CH=CH-N(CH₃)₂, the morpholino, nitro, SO₃H groups,
the groups : 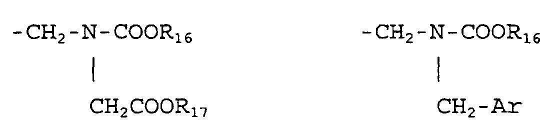
R₁₆ and R₁₇ being selected from among the (C₁-C₆)alkyl groups and Ar being a C₆-C₁₄-aryl group,
with the exception of compounds wherein either R₁, R₂, R₃, R₄, R₅, R₆, R₇ = H or R₁, R₃, R₄, R₅, R₆, R₇ = H and R₂ = Br, or R₁, R₂, R₄, R₅, R₆, R₇ = H and R₃ = OCH₃, or R₁, R₂, R₃, R₄, R₆, R₇ = H and R₅ = OH or OCH₃, or R₁ = NO₂ and R₂, R₃, R₄, R₅, R₆, R₇ = H,
and the addition salts thereof with pharmaceutically acceptable acids.
